# EUROPEAN PATENT APPLICATION

(11) **EP 2 390 251 A1**
(43) Date of publication of application: **30.11.2011**
(21) Application number: 10733193.6
(22) Date of filing: 07.01.2010
(51) Int. Cl.: C07D 405/04, C07D 417/04, C07D 405/14, C07D 417/14, A61K 31/517, A61P 35/00, A61P 17/06

(54) **OPTICAL PURE QUINAZOLINE COMPOUNDS**

(30) Priority: 23.01.2009 CN 200910008526
(71) Applicant: Cen, Junda, Lianyungang, Jiangsu 222047 (CN)
(72) Inventor: TANG, Jiadeng, Shanghai 200437 (CN); WU, Xuesong, Shanghai 200437 (CN)
(74) Representative: Dilg, Haeusler, Schindelmann Patentanwaltsgesellschaft mbH
(86) International application number: PCT/CN2010/000027
(87) International publication number: WO 2010/083720

(57) **Abstract**

Optical pure quinazoline compounds, particularly compounds of general formula (I), wherein R1 and Y are defined as in the specification, preparation methods for them, pharmaceutical compositions containing them and their uses are provided. Compounds of general formula (VII), which are intermediates in the synthesis of the compounds of general formula (I), wherein Ar, R2, R3, m, n, T, and the carbon atom with * are defined as in the specification, are also provided.

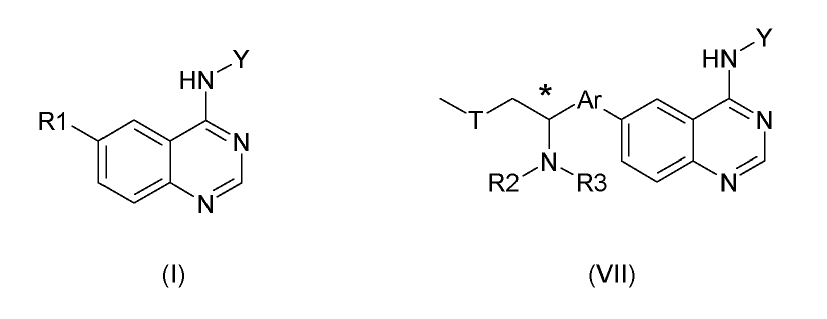

## Description

### FIELD OF THE INVENTION

The present invention relates to a series of novel optical pure quinazoline compounds, a process for their preparation, a pharmaceutical composition containing the same and uses thereof. The present invention also relates to an intermediate in the synthesis of the novel optical pure quinazoline compounds.

### BACKGROUND OF THE INVENTION

Protein-tyrosine kinases catalyze the phosphorylation of specific tyrosyl residues in various proteins which are relevant to regulating the growth and differentiation of the cell. Protein-tyrosine kinase can be generally divided into receptor kinases (such as EGFr, c-erbB-2, c-met, tie-2, PDGFr, FGFr) and non-receptor kinases (such as c-src, lck, zap70). It has shown that many protein-tyrosine kinases could be activated non-properly or uncontrollably, that is, for example, the anomalous protein-tyrosine kinase activation caused by over-expression or mutation will cause uncontrollable cell production.

Abnormal activity of protein-tyrosine kinases, such as c-erbB-2, c-src, c-met, EGFr, PDGFr, is related to human malignant tumor. For example, the elevated activity of EGFr is relevant to non-small cell lung cancer, bladder cancer, and cancer in head or neck. The elevated activity of c-erbB-2 is relevant for breast, ovarian, stomach and pancreas cancer. Therefore, inhibiting protein-tyrosine kinases can provide treatment for the foregoing cancers. Anomalous protein-tyrosine kinases activity is also related to other diseases, such as fiber degeneration, psoriasis, atherosclerosis, restenosis, autoimmune diseases, allergies, asthma, etc. It has been shown that these diseases can be controlled by receptor-tyrosine kinase interaction.

Chinese patent 99803887.3 disclosed aseries of compounds which have inhibitory activity for protein-tyrosine kinases. Chinese patent 20081000815 also disclosed a series of new quinazoline compounds which are all racemic. No research has been performed on their optically pure enantiomers.

### SUMMARY OF THE INVENTION

The present invention provides a series of optical pure quinazoline compounds shown in general formula (I), the process of their preparation and the use thereof.

The present invention also provides a pharmaceutical composition comprising effective dose of the above-mentioned optical pure quinazoline compounds shown in general formula (I), and their use for the treatment of cancers, malignant tumors and psoriasis etc.

The present invention also provides an intermediate which is shown in general formula (VII), in the synthesis of the compound of the general formula (I).

The present invention discloses compounds of general formula (1): 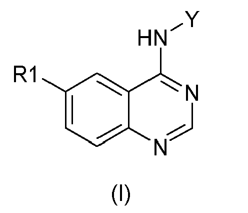 wherein R1 represents 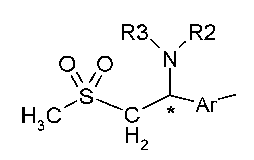 wherein Ar is selected from substituted or unsubstituted furan or thiazole, said substituents being selected from halogen atoms, C₁₋₄ alkyl or C₁₋₄ alkoxy, and the number of the substituents being 1 or 2; and
R2 and R3 are independently selected from (1) hydrogen, (2) alkyl, (3) alkenyl, (4) alkynyl, (5) alkoxy, (6) alkoxy alkyl, (7) cycloalkyl, or (8) cycloalkyl alkyl;
Y is selected from phenyl or 1H-indazolyl, which is optionally substituted with R4, R5; wherein R4 is selected from benzyl, halo-, dihalo- or trihalo-benzyl, benzyloxy, halo-, dihalo- or trihalo-benzyloxy; and R5 is selected from hydrogen, hydroxy, halogen atoms, C₁₋₄ alkyl, C₁₋₄ alkoxy, amino, cyano or trifluoromethyl.

The carbon atom with * is a chiral carbon atom, and exists in the form of a single enantiomer or rich in a kind of enantiomer of (R) or (S).

In a preferred embodiment of this invention, Ar is selected from unsubstituted furan or thiazole, preferably unsubstituted furan.

In another preferred embodiment of this invention, R2 and R3 are independently selected from: (1) hydrogen, (2) C₁₋₄ alkyl, (3) C₂₋₅ alkenyl, (4) C₁₋₄ alkoxy, (5) C₁₋₄ alkoxy C₁₋₄ alkyl, (6) C₃₋₈ cycloalkyl, or (7) C₃₋₈ cycloalkyl-C₁₋₄ alkyl.

In another preferred embodiment of this invention, R4 is selected from benzyl, halo-benzyl, halo-benzyloxy, preferably halo-benzyl or halo-benzyloxy; and R5 is selected from hydrogen, halogen atoms, C₁₋₄ alkyl or C₁₋₄ alkoxy.

In another preferred embodiment of this invention, carbon atom with * exists in the form of a single enantiomer or rich in an enantiomer of (R) or (S). When it exists in the form rich in (R) or (S), preferably the content of enantiomer of (R) or (S) is ≥ 90%.

In an embodiment of this invention, the preferred compounds comprise:
(R)-N-(4-(3-fluorobenzyl-oxy)-3-chlorophenyl)-6-(5-(1-(amino)-2-(methylsulfonyl)ethyl)-furan-2-yl)quinazolin-4-amine; (compound 105)
(S)-N-(4-(3-fluorobenzyl-oxy)-3-chlorophenyl)-6-(5-(1-(amino)-2-(methylsulfonyl)ethyl)-furan-22-yl)quinazolin-4-amine; (compound 92)
(R)-N-(4-(3-fluorobenzyl-oxy)-3-chlorophenyl)-6-(5-(1-(methylamino)-2-(methylsulfonyl)ethyl)-furan-2-yl)quinazolin-4-amine; (compound 106)
(R)-N-(4-(3-fluorobenzyl-oxy)-3-chlorophenyl)-6-(5-(1-(ethylamino)-2-(methylsulfonyl)ethyl)-furan-2-yl)quinazolin-4-amine; (compound 107)
(R)-N-(4-(3-fluorobenzyl-oxy)-3-chlorophenyl)-6-(5-(1-(propylamino)-2-(methylsulfonyl)ethyl)-furan-2-yl)quinazolin-4-amine; (compound 108)
(R)-N-(4-(3-fluorobenzyl-oxy)-3-chlorophenyl)-6-(5-(1-(cyclopropyl-methyl-amino)-2-(methylsulfonyl)ethyl)-furan-2-yl)quinazolin-4-amine; (compound 109)
(R)-N-(4-(3-fluorobenzyl-oxy)-3-chlorophenyl)-6-(5-(1-(N,N-dimethyl-amino)-2-(methylsulfonyl) ethyl)-furan-2-yl)quinazolin-4-amine; (compound 110)
(R)-N-(4-(3-fluorobenzyl-oxy)-3-chlorophenyl)-6-(5-(1-(N,N-diethyl-amino)-2-(methylsulfonyl) ethyl)-furan-2-yl)quinazolin-4-amine; (compound 111)
(R)-N-(4-(3-fluorobenzyl-oxy)-3-chlorophenyl)-6-(5-(1-(N,N-dipropyl-amino)-2-(methylsulfonyl) ethyl)-furan-2-yl)quinazolin-4-amine; (compound 112)
(R)-N-(4-(3-fluorobenzyloxy)-3-chlorophenyl)-6-(5-(1-(N-methyl, N-ethyl-amino)-2-(methylsulfonyl)ethyl)-furan-2-yl)quinazolin-4-amine; (compound 113)
(R)-N-(4-(3-fluorobenzyl-oxy)-3-chlorophenyl)-6-(5-(1-(allyl-amino)-2-(methylsulfonyl)ethyl)-furan-2-yl)quinazolin-4-amine; (compound 114)
(R)-N-(4-(3-fluorobenzyl-oxy)-3-chlorophenyl)-6-(5-(1-(propargyl-amino)-2-(methylsulfonyl) ethyl)-furan-2-yl)quinazolin-4-amine; (compound 115)
(S)-N-(4-(3-fluorobenzyl-oxy)-3-chlorphenyl)-6-(5-(1-(methylamino)-2-(methylsulfonyl)ethyl)-furan-2-yl)quinazolin-4-amine; (compound 93)
(S)-N-(4-(3-fluorobenzyl-oxy)-3-chlorophenyl)-6-(5-(1-(ethylamino)-2-(methylsulfonyl)ethyl)-furan-2-yl)quinazolin-4-amine; (compound 94)
(S)-N-(4-(3-fluorobenzyl-oxy)-3-chlorophenyl)-6-(5-(1-(propylamino)-2-(methylsulfonyl)ethyl)-furan-2-yl)quinazolin-4-amine; (compound 95)
(S)-N-(4-(3-fluorobenzyl-oxy)-3-chlorophenyl)-6-(5-(1-(cyclopropyl-methyl-amino)-2-(methylsulfonyl)ethyl)-furan-2-yl)quinazolin-4-amine; (compound 96)
(S)-N-(4-(3-fluorobenzyloxy)-3-chlorophenyl)-6-(5-(1-(N,N-dimethyl-amino)-2-(methylsulfonyl) ethyl)-furan-2-yl)quinazolin-4-amine; (compound 97)
(S)-N-(4-(3-fluorobenzyl-oxy)-3-chlorophenyl)-6-(5-(1-(N,N-diethylamino)-2-(methyl-sulfonyl) ethyl)-furan-2-yl)quinazolin-4-amine; (compound 98)
(S)-N-(4-(3-fluorobenzyl-oxy)-3-chlorophenyl)-6-(5-(1-(N,N-dipropyl-amino)-2-(methylsulfonyl) ethyl)-furan-2-yl)quinazolin-4-amine; (compound 99)
(S)-N-(4-(3-fluorobenzyl-oxy)-3-chlorophenyl)-6-(5-(1-(N-methyl,N-ethyl-amino)-2-(methylsulfonyl)ethyl)-furan-2-yl)quinazolin-4-amine; (compound 100)
(S)-N-(4-(3-fluorobenzyl-oxy)-3-chlorophenyl)-6-(5-(1-(allyl-amino)-2-(methylsulfonyl)ethyl)-furan-2-yl)quinazolin-4-amine; (compound 101)
(S)-N-(4-(3-fluorobenzyl-oxy)-3-chlorophenyl)-6-(5-(1-(propargyl-amino)-2-(methylsulfonyl) ethyl)-furan-2-yl)quinazolin-4-amine; (compound 102)
(S)-N-(1-(3-fluorobenzyl)-1H-indazol-5-yl)-6-(5-(1-(amino)-2-(methylsulfonyl)ethyl)-furan-2-yl) quinazolin-4-amine; (compound 144)
(S)-N-(1-(3-fluorobenzyl)-1H-indazol-5-yl)-6-(5-(1-(methylamino)-2-(methylsulfonyl)ethyl)-furan-2-yl)quinazolin-4-amine; (compound 145)
(S)-N-(1-(3-fluorobenzyl)-1H-indazol-5-yl)-6-(5-(1-(ethylamino)-2-(methylsulfonyl)ethyl)-furan-2-yl)quinazolin-4-amine; (compound 146)
(S)-N-(1-(3-fluorobenzyl)-1H-indazol-5-yl)-6-(5-(1-(propylamino)-2-(methylsulfonyl)ethyl)-furan-2-yl)quinazolin-4-amine; (compound 147)
(S)-N-(1-(3-fluorobenzyl)-1H-indazol-5-yl)-6-(5-(1-(N,N-dimethylamino)-2-(methylsulfonyl) ethyl)-furan-2-yl)quinazolin-4-amine; (compound 149)
(S)-N-(1-(3-fluorobenzyl)-1H-indazol-5-yl)-6-(5-(1-(cyclopropyl-methyl-amino)-2-(methylsulfonyl)ethyl)-furan-2-yl)quinazolin-4-amine; (compound 148)
(S)-N-(1-(3-fluorobenzyl)-1H-indazol-5-yl)-6-(5-(1-(allyl-amino)-2-(methylsulfonyl)ethyl)-furan-2-yl)quinazolin-4-amine; (compound 153); and
(S)-N-(1-(3-fluorobenzyl)-1H-indazol-5-yl)-6-(5-(1-(propargyl-amino)-2-(methylsulfonyl)ethyl)-furan-2-yl)quinazolin-4-amine (compound 154).

Within the present invention,
- "rich in an enantiomer" refers to the content of enantiomer of (R) or (S) is ≥60%;
- "alkyl" refers to branched or straight-chain saturated aliphatic hydrocarbon groups; preferably branched or straight-chain saturated aliphatic alkyl groups with 1 to 4 carbon atoms, such as methyl, ethyl, propyl, isopropyl, butyl, tert-butyl, isobutyl, etc.;
- "alkenyl" refers to branched, straight-chain or non-aromatic hydrocarbon ring group which contains at least one carbon-carbon double bonds, such as vinyl, propenyl, allyl, butenyl, cyclohexene, etc.;
- "alkynyl" refers to branched, straight-chain or cyclic hydrocarbon group which contains at least one carbon-carbon triple bond, such as acetenyl, propinyl, butynyl, 3-methyl-butynyl, butynyl, propargyl, etc.;
- "cycloalkyl" refers to saturated aliphatic hydrocarbon group which contains monocyclic ring, preferably cycloalkyl which contains 3-8 carbon atoms, such as cyclopropyl, methyl-cyclopropyl, 2,2-dimethyl-cyclobutyl, ethyl-cyclopentyl, cyclohexyl, etc.;
- "alkoxy" refers to a group in which straight or branched chain alkyl is connected to oxygen atoms, such as methoxy, ethoxy, propoxy, isopropoxy, butoxy, isobuoxy, tert-butoxy, etc.; and
- "halogen atoms" refer to fluorine, chlorine, bromine, iodine atoms.

The invention also provides the preparation process of compounds in general formula (I), which comprising the following step:
(1) reacting the compound of general formula (II) with tert-butylsulfinamide to prepare the compound of general formula(III); 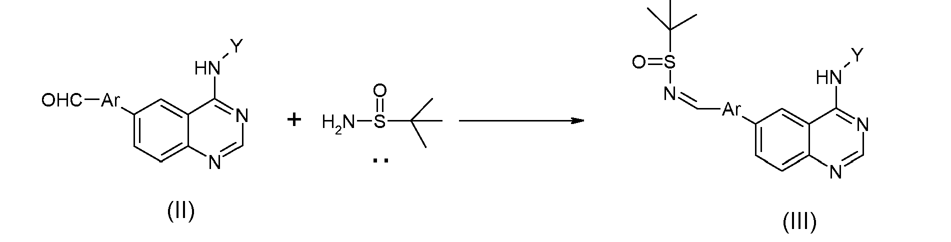
(2) reacting the compound of general formula (III) with the compound of general formula (IV) to prepare the compound of general formula (V); 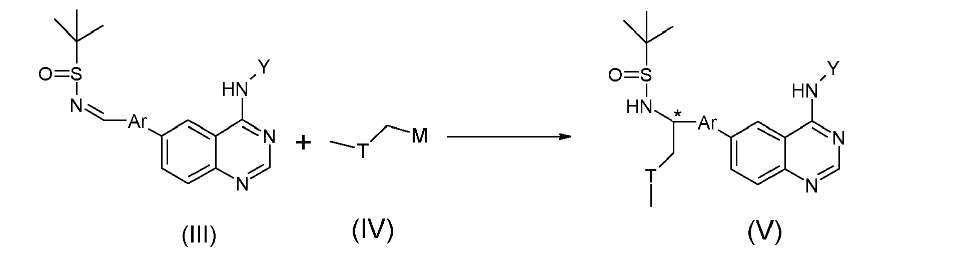
(3) generating the compound of general formula (VI) from the compound of general formula(V)in acid condition. 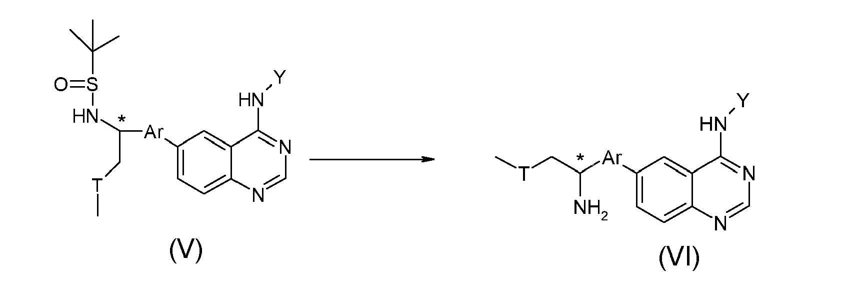
(4) reacting the compound of general formula (VI) with reagent R2-L or R3-L to prepare the compound of general formula (VII); and 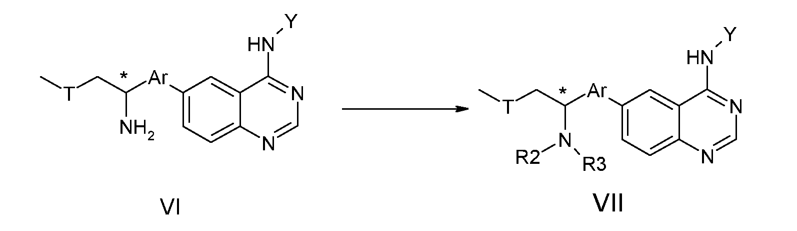
(5) reacting the compound of general formula (VII) with an oxidant to prepare the compound of general formula (I); 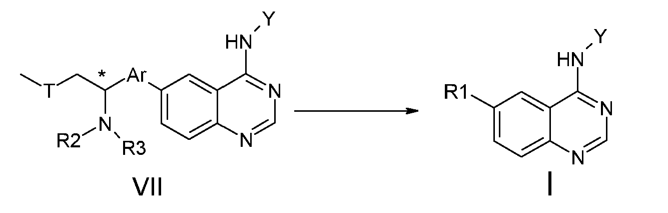 wherein: R1, Y, Ar, R2, R3, and the carbon atom with * are as defined in formula (I);
   T is s sulfur atom or sulfinyl;
   Ttrt-butylsulfinamide is optically pure, and exists in the form of a single enantiomer or rich in an enantiomer of (R) or (S);
   M is an alkali metal ion or a halogen atomsated-alkaline earth metal ion, selected from Li⁺, Na⁺, K⁺, [MgCl]⁺ or [MgBr]⁺; and
   L is a leaving group, selected from halogen atoms or a sulfonyloxyl group.

For preparing compounds of general formula (III), the reaction is carried out in the presence of metallic reagents comprising tetraethoxy-titanium, tetra-isopropyl- titanate etc., and preferably tetra-isopropyl-titanate. The reaction temperature is 0-100°C, and preferably 0-50°C.

For preparing compounds of general formula (VI), the reaction is carried out under acidic conditions. The acid is selected from hydrochloric acid, sulfuric acid, nitric acid, phosphoric acid, trifluoroacetic acid or the mixture of these acids, and preferably hydrochloric acid.

For preparing compounds of general formula (I), oxidation of sulfur atom or sulfinyl to sulfonyl is well known by one skilled in the art. The oxidant is selected from chloroperoxybenzoic acid, peracetic acid, hydrogen peroxide or potassium monopersulfate, preferably potassium monopersulfate.

The invention also provides the preparation process of compounds in general formula (I), comprising the following steps:
(1) reacting the compound of general formula(II)with tert-butylsulfinamide to prepare the compound of general formula (III); 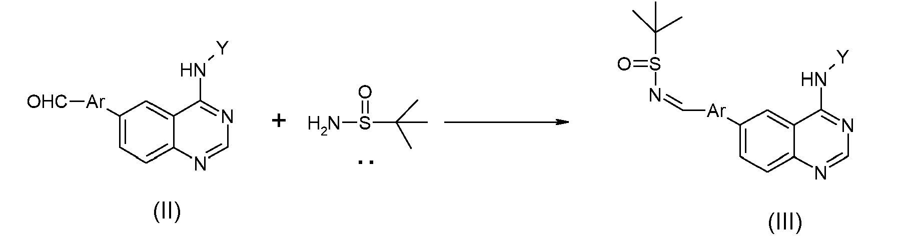
(2) reacting the compound of general formula (III) with the compound of general formula (A) to prepare the compound of general formula (B); 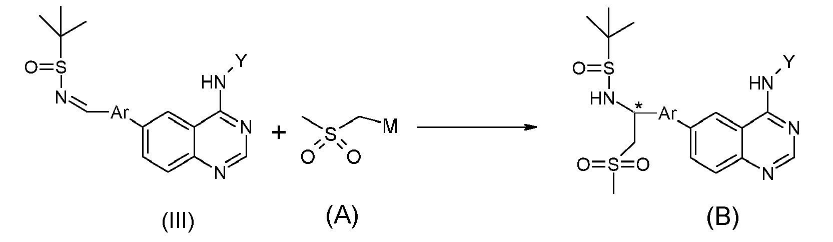
(3) generating the compound of general formula (C) from the compound of general formula (B) in acid condition. 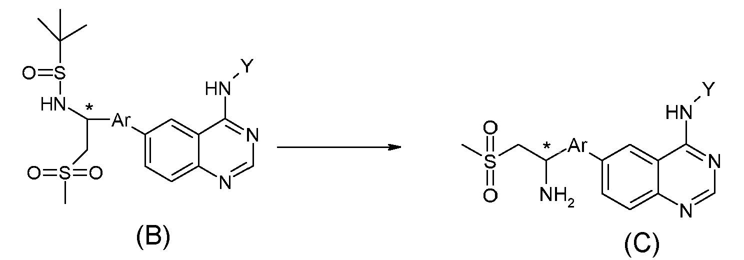
(4) reacting the compound of general formula (C) with reagent R2-L or R3-L to prepare the compound of general formula (I); 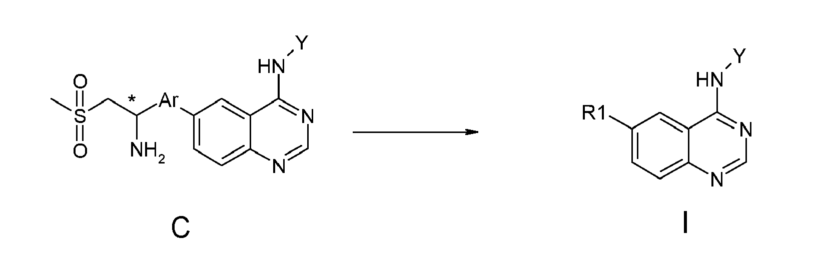
wherein: R1, Y, Ar, R2, R3, and the carbon atom with * are as defined in general formula (I); and tert-butylsulfinamide, M, and L are as defined above.

For preparing compounds of general formula (I), L represents leaving group well-known by a skilled person, such as halogen atoms (e.g., fluorine, chlorine, bromine, iodine, preferably bromine and iodine); a sulfonyloxyl group (e.g., methylsulfonyloxyl, toluenesulfonyloxyl).

For preparing compounds of general formula (C), the reaction is carried out under acidic condition, and the acid is selected from hydrochloric acid, sulfuric acid, nitric acid, phosphoric acid, trifluoroacetic acid or the mixture of these acids, and preferably hydrochloric acid.

The reaction of step 4 is carried out under alkaline condition, and the alkaline is selected from inorganic base (such as sodium bicarbonate, sodium carbonate, potassium carbonate, sodium hydroxide, potassium hydroxide, etc.) or organic base (such as ethylamine, triethylamine, diisopropylethylamine, etc.).

The invention also provides the intermediate compounds represented by general formula (VII), which are the key intermediates for synthesis of the compounds of general formula (I). 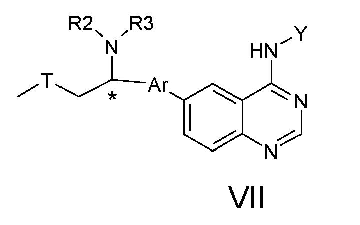 wherein Y, Ar, R2, R3, and the carbon atom with * are as defined in formula (I); and T is sulfur atom or sulfinyl.

In the present invention, preferably compounds of general formula (VII) comprise:
(R)-N-(4-(3-fluorobenzyl-oxy)-3-chlorophenyl)-6-(5-(1-(amino)-2-(methylthio)ethyl)-furan-2-yl) quinazolin-4-amine; (compound 40)
(R)-N-(4-(3-fluorobenzyl-oxy)-3-chlorophenyl)-6-(5-(1-(methylamino)-2-(methylthio)ethyl)-furan-2-yl)quinazolin-4-amine; (compound 41)
(R)-N-(4-(3-fluorobenzyl-oxy)-3-chlorophenyl)-6-(5-(1-(ethylamino)-2-(methylthio)ethyl)-furan-2-yl)quinazolin-4-amine; (compound 42)
(R)-N-(4-(3-fluorobenzyl-oxy)-3-chlorophenyl)-6-(5-(1-(propylamino)-2-(methylthio)ethyl)-furan-2-yl)quinazolin-4-amine; (compound 43)
(R)-N-(4-(3-fluorobenzyl-oxy)-3-chlorophenyl)-6-(5-(1-(cyclopropyl-methyl-amino)-2-(methylthio)ethyl)-furan-2-yl)quinazolin-4-amine; (compound 44)
(R)-N-(4-(3-fluorobenzyl-oxy)-3-chlorophenyl)-6-(5-(1-(N,N-dimethylamino)-2-(methylthio) ethyl)-furan-2-yl)quinazolin-4-amine; (compound 45)
(R)-N-(4-(3-fluorobenzyl-oxy)-3-chlorophenyl)-6-(5-(1-(N,N-diethylamino)-2-(methylthio)ethyl) -furan-2-yl)quinazolin-4-amine; (compound 46)
(R)-N-(4-(3-fluorobenzyl-oxy)-3-chlorophenyl)-6-(5-(1-(N,N-dipropyl-amino)-2-(methylthio) ethyl)-furan-2-yl)quinazolin-4-amine; (compound 47)
(R)-N-(4-(3-fluorobenzyl-oxy)-3-chlorophenyl)-6-(5-(1-(N-methyl,N-ethyl-amino)-2-(methylthio) ethyl)-furan-2-yl)quinazolin-4-amine; (compound 48)
(R)-N-(4-(3-fluorobenzyl-oxy)-3-chlorophenyl)-6-(5-(1-(allyl-amino)-2-(methylthio)ethyl)-furan-2-yl)quinazolin-4-amine; (compound 49)
(R)-N-(4-(3-fluorobenzyl-oxy)-3-chlorophenyl)-6-(5-(1-(propargylamino)-2-(methylthio)ethyl)-furan-2-yl)quinazolin-4-amine; (compound 50)
(R)-N-(4-(3-fluorobenzyl-oxy)-3-chlorophenyl)-6-(5-(1-(methylamino)-2-(methylsulfinyl)ethyl)-furan-2-yl)quinazolin-4-amine; (compound 80)
(R)-N-(4-(3-fluorobenzyl-oxy)-3-chlorophenyl)-6-(5-(1-(ethylamino)-2-(methylsulfinyl)ethyl)-furan-2-yl)quinazolin-4-amine; (compound 81)
(R)-N-(4-(3-fluorobenzyl-oxy)-3-chlorophenyl)-6-(5-(1-(propylamino)-2-(methylsulfinyl)ethyl)-furan-2-yl) quinazolin-4-amine; (compound 82)
(R)-N-(4-(3-fluorobenzyl-oxy)-3-chlorophenyl)-6-(5-(1-(cyclopropyl-methyl-amino)-2-(methylsulfinyl)ethyl)-furan-2-yl)quinazolin-4-amine; (compound 83)
(R)-N-(4-(3-fluorobenzyl-oxy)-3-chlorophenyl)-6-(5-(1-(N,N-dimethyl-amino)-2-(methylsulfinyl) ethyl)-furan-2-yl)quinazolin-4-amine; (compound 84)
(R)-N-(4-(3-fluorobenzyl-oxy)-3-chlorophenyl)-6-(5-(1-(N,N-diethylamino)-2-(methylsulfinyl) ethyl)-furan-2-yl)quinazolin-4-amine; (compound 85)
(R)-N-(4-(3-fluorobenzyl-oxy)-3-chlorophenyl)-6-(5-(1-(N,N-dipropyl-amino)-2-(methylsulfinyl) ethyl)-furan-2-yl)quinazolin-4-amine; (compound 86)
(R)-N-(4-(3-fluorobenzyl-oxy)-3-chlorophenyl)-6-(5-(1-(N-methyl,N-ethyl-amino)-2-(methylsulfinyl)-furan-2-yl)quinazolin-4-amine; (compound 87)
(R)-N-(4-(3-fluorobenzyl-oxy)-3-chlorophenyl)-6-(5-(1-(allyl-amino)-2-(methylsulfinyl)ethyl)-furan-2-yl)quinazolin-4-amine; (compound 88)
(R)-N-(4-(3-fluorobenzyl-oxy)-3-chlorophenyl)-6-(5-(1-(propargylamino)-2-(methylsulfinyl) ethyl)-furan-2-yl)quinazolin-4-amine; (compound 89)
(S)-N-(4-(3-fluorobenzyl-oxy)-3-chlorophenyl)-6-(5-(1-(amino)-2-(methylthio)ethyl)-furan-2-yl) quinazolin-4-amine; (compound 1)
(S)-N-(4-(3-fluorobenzyl-oxy)-3-chlorophenyl)-6-(5-(1-(methylamino)-2-(methylthio)ethyl)-furan-2-yl)quinazolin-4-amine; (compound 2)
(S)-N-(4-(3-fluorobenzyl-oxy)-3-chlorophenyl)-6-(5-(1-(ethylamino)-2-(methyl-thio)ethyl)-furan -2-yl)quinazolin-4-amine; (compound 3)
(S)-N-(4-(3-fluorobenzyl-oxy)-3-chlorophenyl)-6-(5-(1-(propylamino)-2-(methylthio)ethyl)-furan-2-yl)quinazolin-4-amine; (compound 4)
(S)-N-(4-(3-fluorobenzyl-oxy)-3-chlorophenyl)-6-(5-(1-(cyclopropyl-methyl-amino)-2-(methylthio)ethyl)-furan-2-yl) quinazolin-4-amine; (compound 5)
(S)-N-(4-(3-fluorobenzyl-oxy)-3-chlorophenyl)-6-(5-(1-(N,N-dimethyl-amino)-2-(methylthio) ethyl)-furan-2-yl)quinazolin-4-amine; (compound 6)
(S)-N-(4-(3-fluorobenzyl-oxy)-3-chlorophenyl)-6-(5-(1-(N,N-diethylamino)-2-(methylthio)ethyl) -furan-2-yl)quinazolin-4-amine; (compound 7)
(S)-N-(4-(3-fluorobenzyl-oxy)-3-chlorophenyl)-6-(5-(1-(N,N-dipropyl-amino)-2-(methylthio) ethyl)-furan-2-yl)quinazolin-4-amine; (compound 8)
(S)-N-(4-(3-fluorobenzyl-oxy)-3-chlorophenyl)-6-(5-(1-(N-methyl,N-ethyl-amino)-2-(methylthio) ethyl) furan-2-yl)quinazolin-4-amine; (compound 9)
(S)-N-(4-(3-fluorobenzyl-oxy)-3-chlorophenyl)-6-(5-(1-(allyl-amino)-2-(methylthio)ethyl)-furan-2-yl)quinazolin-4-amine; (compound 10)
(S)-N-(4-(3-fluorobenzyl-oxy)-3-chlorophenyl)-6-(5-(1-(propargylamino)-2-(methylthio)ethyl)-furan-2-yl)quinazolin-4-amine; (compound 11)
(S)-N-(1-(3-fluorobenzyl)-1H-indazol-5-yl)-6-(5-(1-(methylamino)-2-(methylthio)ethyl)-furan-2-yl)quinazolin-4-amine; (compound 28)
(S)-N-(1-(3-fluorobenzyl)-1H-indazol-5-yl)-6-(5-(1-(ethylamino)-2-(methylthio)ethyl)-furan-2-yl) quinazolin-4-amine; (compound 29)
(S)-N-(1-(3-fluorobenzyl)-1H-indazol-5-yl)-6-(5-(1-(propyl-amino)-2-(methylthio)ethyl)-furan-2-yl)quinazolin-4-amine; (compound 30)
(S)-N-(1-(3-fluorobenzyl)-1H-indazol-5-yl)-6-(5-(1-(N,N-dimethyl-amino)-2-(methylthio)ethyl)-furan-2-yl)quinazolin-4-amine; (compound 32)
(S)-N-(1-(3-fluorobenzyl)-1H-indazol-5-yl)-6-(5-(1-(cyclopropyl-methyl-amino)-2-(methylthio) ethyl)-furan-2-yl)quinazolin-4-amine; (compound 31)
(S)-N-(1-(3-fluorobenzyl)-1H-indazol-5-yl)-6-(5-(1-(N-methyl,N-ethyl-amino)-2-(methylthio) ethyl)-furan-2-yl)quinazolin-4-amine; (compound 35)
(S)-N-(1-(3-fluorobenzyl)-1H-indazol-5-yl)-6-(5-(1-(allyl-amino)-2-(methylthio)ethyl)-furan-2-yl) quinazolin-4-amine; (compound 36)
(S)-N-(1-(3-fluorobenzyl)-1H-indazol-5-yl)-6-(5-(1-(propargylamino)-2-(methylthio)ethyl)-furan-2-yl)quinazolin-4-amine; (compound 37)
(S)-N-(4-(3-fluorobenzyl-oxy)-3-chlorophenyl)-6-(5-(1-(methylamino)-2-(methylsulfinyl)ethyl)-furan-2-yl)quinazolin-4-amine; (compound 67)
(S)-N-(4-(3-fluorobenzyl-oxy)-3-chlorophenyl)-6-(5-(1-(ethylamino)-2-(methylsulfinyl)ethyl)-furan-2-yl)quinazolin-4-amine; (compound 68)
(S)-N-(4-(3-fluorobenzyl-oxy)-3-chlorophenyl)-6-(5-(1-(propylamino)-2-(methylsulfinyl)ethyl)-furan-2-yl)quinazolin-4-amine; (compound 69)
(S)-N-(4-(3-fluorobenzyl-oxy)-3-chlorophenyl)-6-(5-(1-(cyclopropyl-methyl-amino)-2-(methylsulfinyl)ethyl)-furan-2-yl)quinazolin-4-amine; (compound 70)
(S)-N-(4-(3-fluorobenzyl-oxy)-3-chlorophenyl)-6-(5-(1-(N,N-dimethyl-amino)-2-(methylsulfinyl) ethyl)-furan-2-yl)quinazolin-4-amine; (compound 71)
(S)-N-(4-(3-fluorobenzyl-oxy)-3-chlorophenyl)-6-(5-(1-(N,N-diethyl-amino)-2-(methylsulfinyl) ethyl)-furan-2-yl)quinazolin-4-amine; (compound 72)
(S)-N-(4-(3-fluorobenzyl-oxy)-3-chlorophenyl)-6-(5-(1-(N,N-dipropyl-amino)-2-(methylsulfinyl) ethyl)-furan-2-yl)quinazolin-4-amine; (compound 73)
(S)-N-(4-(3-fluorobenzyl-oxy)-3-chlorophenyl)-6-(5-(1-(N-methyl,N-ethyl-amino)-2-(methylsulfinyl)ethyl)-furan-2-yl)quinazolin-4-amine; (compound 74)
(S)-N-(4-(3-fluorobenzyl-oxy)-3-chlorophenyl)-6-(5-(1-(allyl-amino)-2-(methylsulfinyl)ethyl)-furan-2-yl)quinazolin-4-amine; (compound 75); and
(S)-N-(4-(3-fluorobenzyl-oxy)-3-chlorophenyl)-6-(5-(1-(propargyl-amino)-2-(methylsulfinyl) ethyl)-furan-2-yl)quinazolin-4-amine; (compound 76).

The invention also provides a pharmaceutical composition comprising the above-mentioned therapeutically effective amount of the compound of general formula (I) or pharmaceutically acceptable salts thereof and pharmaceutically acceptable carriers.

The invention also provides the use of the compounds of general formula (I) or pharmaceutically acceptable salts thereof for the preparation of a medicament for the treatment of diseases associated with regulating c-erbB-2 and/or EGF-R protein tyrosine kinase activity (i.e. the compounds of general formula (I) or pharmaceutically acceptable salts thereof for use in the treatment of diseases associated with regulating c-erbB-2 and/or EGF-R protein tyrosine kinase activity).

The invention also provides the use of the pharmaceutical composition comprising the compounds of general formula (I) or pharmaceutically acceptable salts thereof for the preparation of a medicament for the treatment of diseases associated with regulating c-erbB-2 and/or EGF-R protein tyrosine kinase activity.

The invention also provides the use of the compounds of general formula (I) or pharmaceutically acceptable salts thereof in the preparation of a medicament for the treatment of cancer and malignant tumors. The invention also provides the use of the compounds of general formula (I) or pharmaceutically acceptable salts thereof in the preparation of a medicament for the treatment of psoriasis.

The invention also provides the use of the pharmaceutical composition comprising the compounds of general formula (I) or pharmaceutically acceptable salts thereof in the preparation of a medicament for the treatment of cancer and malignant tumors. The invention also provides the use of the pharmaceutical composition comprising the compounds of general formula (I) or pharmaceutically acceptable salts thereof in the preparation of a medicament for the treatment of psoriasis.

The pharmaceutical preparations in the present invention can occur as unit dose, with each unit dose containing a predetermined quantity of active ingredient. Such unit dose may contain such as 0.5 mg to 1 g. The specific dosage depends on the disease, route of administration and the patient's age, weight, condition and other factors.

The pharmaceutical preparations can be administrated by any suitable ways, such as oral, rectal, nasal, local or parenteral (including subcutaneous, intramuscular, intravenous or transdermal) administration etc. The above pharmaceutical preparations can be prepared by any methods known in the pharmaceutical field, such as by mixing active ingredients with a carrier or an excipient.

The compounds or pharmaceutically acceptable salts thereof of the invention can be administrated alone or in combination with other therapeutic agents for treatment of the above diseases. Administration in combination with other chemotherapeutic agents, hormones or antibody drugs should be considered, especially in anti-tumor therapy.

### DETAILED DESCRIPTION OF THE INVENTION

To describe the present invention in more detail, the following examples are provided. However, the scope of the present invention is not limited to these.

The enantiomer excesses (e.e) in the following embodiment refer to relative amount of each enantiomer. The value is defined as the difference of the relative percentage of two enantiomers. For example, when the percentage of (R) enantiomer is 90%, and the percentage of (S) enantiomer is 10%, then the value of e.e is 80%.

A chiral high performance liquid chromatography (HPLC) was used to measure the enantiomer of each compound, the method is as below:
Column: Daicel AD; mobile phase: n-hexane-ethanol-diethylamine (50:50: 0.1).

### Example 1: Preparation of N-(4-(3-fluorobenzyloxy)-3-chlorophenyl)-6-iodine-quinazolin-4-amine

6-lodine-3H-quinazolin-4-ketone (100g) was added to a 2000 ml flask, dissolved in mixed solvent of thionyl chloride (1000 ml) and N,N-dimethylformamide (20 ml), heated to reflux until the reaction solution was clear and transparent. After removal of thionyl chloride anhydrous toluene was added to the residues and removed under reduced pressure. The process of adding and removing toluene was repeated to remove the remaining thionyl chloride residues.

The intermediate was dissolved in isopropyl alcohol (2000ml), 3-chloro-4-(3-fluorobenzyloxy)-aniline hydrochloride was added, add anhydrous K₂CO₃(150 g) was added with mechanical stirring before the mixture was heated to reflux over night. The reaction solution was cooled to room temperature over night. The precipitation was filtered and washed with water multiple times until the pH of washing solution became neutral. After drying under vacuum, 95 g of product were collected in a pale white solid. m/z(M+1)⁺: 506

### Example 2: Preparation of N-(1-(3-fluorobenzyl)-1H-indazol-5-yl)-6-iodoquinazolin-4-amine

The process was the same as that in example 1, except that 3-chloro-4-(3-fluoro-benzyloxy) -aniline hydrochloride was replaced by 1-(3-fluorobenzyl)-1H-indazol-5-amine hydrochloride. m/z(M+1)⁺: 496.

### Example 3: Preparation of 5-(4-(4-(3-fluorobenzyl-oxy)-3-chlorophenyl-amino)quinazolin -6-yl)-furan-2-aldehyde

The product (50 g) of example 1, 5-boric acid-2-furfural (21 g), Pd(PPh₃)₂Cl₂ (6.2 g), triethylamine (62 ml), and methyl alcohol (1000 ml) were added into the reaction flask, and refluxed for 2h. After the reaction solution was cooled to room temperature, the precipitate was filtered and washed with a small amount of methanol, then dried at 50°C to obtain the 40g subject product in a yellow solid. m/z (M+1)⁺: 473.

### Example 4: Preparation of 5-(4-(1-(3-fluorobenzyl)-1H-indazol-5-yl-amino)quinazolin-6-yl)-furan-2-aldehyde

The process was the same as in example 3, except that the raw material compound of preparation example 1 was replaced by compound of example 2. m/z(M+1)⁺: 464.

### Example 5: Preparation of 2-(4-(4-(3-fluorobenzyloxy)-3-chlorophenyl-amino)quinazolin-6-yl)-thiazole-5-aldehyde

The product (50 g) of example 1, 2-boric acid-5-thiazole aldehyde (21 g), Pd(PPh₃)₂Cl₂(6.2 g), triethylamine (62 ml), and methyl alcohol (1000 ml) were added into the reaction flask and refluxed for 2h. After the reaction solution was cooled to room temperature, the precipitation was filtered and washed with a small amount of methanol, then dried at 50°C to obtain 30g product. m/z(M+1)⁺: 490.

### Example 6: Preparation of 2-(4-(1-(3-fluorobenzyl)-1H-indazol-5-amino)quinazolin-6-yl)-thiazole-5-aldehyde

The process was the same as in example 5, except that the raw material compound of example 1 was replaced by compound of example 2. m/z(M+1)⁺: 480

### Embodiment 1: Preparation of (S)-N-((5-(4-(4-(3-fluorobenzyl-oxy)-3-chlorophenyl-amino) quinazolin-6-yl)-furan-2-yl)methylenyl)-2-methylpropane-2-sulfinamide

The product of example 3 (47.3 g, 0.1 mol), (S)-(-)-2-methyl-2-propanesulfinamide (14.5 g, 0.12 mol), titanium (IV) isopropoxide (tetra-isopropyl titanate) (85 g, 0.3 mol), and anhydrous THF (1000 ml) were added into the reaction flask and reacted at room temperature over night. Then, water (50 ml) and ethyl acetate (500 ml) were added under stirring for 10min, followed by a filtration, and the precipitation was washed with THF for three times. The combined filtrates were dried with anhydrous magnesium sulfate, filtered and concentrated under reduced pressure to dry to obtain 50g solid compound. m/z(M+1)⁺: 577.

### Embodiment 2: Preparation of (R)-N-((5-(4-(4-(3-fluorobenzyl-oxy)-3-chlorophenyl-amino) quinazolin-6-yl)-furan-2-yl)methylenyl)-2-methylpropane-2-sulfinamide

The process was the same as in embodiment 1, except that the raw material (S)-(-)-2-methyl-2-propanesulfinamide was replaced by (R)-(+)-2-methyl-2-propanesulfinamide. m/z(M+1)⁺: 577.

### Embodiment 3: Preparation of (S)-N-((2-(4-(4-(3-fluorobenzyl-oxy)-3-chlorophenyl-amino) quinazolin-6-yl)-thiazole-5-yl)methylenyl)-2-methylpropane-2-sulfinamide

The process was the same as in embodiment 1, except that the raw material compound of example 3 was replaced by the compound of example 5. m/z(M+1)⁺: 594.

### Embodiment 4: Preparation of (R)-N-((2-(4-(4-(3-fluorobenzyl-oxy)-3-chlorophenyl-amino) quinazolin-6-yl)-thiazole-5-yl)methylenyl)-2-methylpropane-2-sulfinamide

The process was the same as in embodiment 3, except that the raw material (S)-(-)-2-methyl-2-propanesulfinamide was replaced by (R)-(+)-2-methyl-2-propanesulfinamide. m/z(M+1)⁺: 594.

### Embodiment 5: Preparation of (S)-N-((5-(4-(1-(3-fluorobenzyl)-1H-indazol-5-amino)quinazolin-6-yl)-furan-2-yl)methylenyl)-2-methylpropane-2-sulfinamide

The process was the same as in embodiment 1, except that the raw material compound of example 3 was replaced by the compound of example 4. m/z(M+1)⁺: 567.

### Embodiment 6: Preparation of (R)-N-((5-(4-(1-(3-fluorobenzyl)-1H-indazol-5-amino)quinazolin-6-yl) furan-2-yl) methylenyl)-2-methylpropane-2- sulfonamide

The process was the same as in embodiment 2, except that the raw material compound of example 3 was replaced by the compound of example 4. m/z(M+1)⁺: 567.

### Embodiment 7: Preparation of (S)-N-((2-(4-(1-(3-fluorobenzyl)-1H-indazol-5-amino)quinazolin-6-yl)-thiazole-5-yl)methylenyl)-2-methylpropane-2-sulfinamide

The process was the same as in embodiment 1, except that the raw material compound of example 3 was replaced by the compound of example 6. m/z(M+1)⁺: 584

### Embodiment 8: Preparation of (R)-N-((2-(4-(1-(3-fluorobenzyl)-1H-indazol-5-amino)quinazolin-6-yl) thiazole-5-yl)methylenyl)-2-methylpropane-2-sulfinamide

The process was the same as in embodiment 2, except that the raw material compound of example 3 was replaced by the compound of example 6. m/z(M+1)⁺: 584.

### Embodiment 9: Preparation of (S)-N-(4-(3-fluorobenzyloxy)-3-chlorophenyl)-6-(5-(1-(amino) -2-(methylthio)ethyl)-furan-2-yl)quinazolin-4-amine

Methylthio-methyl magnesium chloride/THF solution (0.3 mol) was added into the reaction flask and the reaction solution was cooled below -80°C. Then, the solution of the product of embodiment 1 (57.6 g, 0.1 mol) in anhydrous THF (200 ml) was added rapidly to the flask at-80°C, and stirring for 10min at the same temperature was performed. Saturated saline (3000 ml) was decanted into the reaction solution, and ethyl acetate (2000 ml) was added to extract the product. The organic layer was washed with saturated saline (2000 ml), and dried with anhydrous magnesium sulfate followed by a filtration to obtain 50 g yellow solid by concentrating the filtrate under reduced pressure.

The yellow solid from the above step was dissolved in THF (1000 ml) and the pH of the solution was adjusted to 1 with HCl-ethanol. The solution was stirred for 2 hours at room temperature. After the pH of the solution was adjusted to 9 with ammonia, saturated saline (2000 ml) and ethyl acetate (1500 ml) were added to the solution. The organic layer was separated and dried with anhydrous magnesium sulfate followed by a filtration and concentrated under reduced pressure The residue was purified by silica-gel column chromatography (eluent: ethyl acetate-ethyl acetate/THF=10/1), and the fraction in need was collected, concentrated to obtain 30g product designated as compound 1. m/z(M+1)⁺: 535.

### Embodiment 10: Preparation of (S)-N-(4-(3-fluorobenzyl-oxy)-3-chlorophenyl)-6-(5-(1-(methyl-amino)-2-(methylthio)ethyl)-furan-2-yl)quinazolin-4-amine

Method A: the product (2.0 g) obtained in embodiment 9, iodomethane (0.5 g), and triethylamine (0.7 g) were dissolved in THF (150 ml), heated to reflux for 2h, and then cooled to room temperature. To the reaction solution, saturated saline and ethyl acetate were added. The separated organic layer was washed twice with saturated saline, dried with anhydrous magnesium sulfate. After the filtration and concentration under reduced pressure, the residue was purified by silica-gel column chromatography (chloroform/methanol = 100:1), to obtain the product (1.5 g) designated as compound 2.

Method B: the product (2.0 g) of embodiment 9 was dissolved in DMSO (50 ml). Then formaldehyde (6 ml) and formic acid (3 ml) were added and the solution was stirred over night at room temperature. The reaction solution was mixed with ice water (500 ml), and the solid was filtered and collected. After dissolving with THF, the solution of the solid collection was purified by silica-gel column chromatography to obtain 4.2 g product designated as compound 2. m/z(M+1)+: 549.

According to the preparation methods of embodiment 10, the compound obtained in embodiment 9, as the starting material, was used to react with reagents to prepare the following compound:

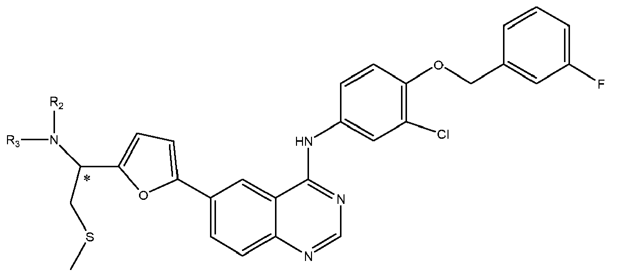

Remark: the carbon atom with * is (S)-configuration

| **Number** | **Reagent** | **R2 =** | **R3 =** | **m/z(M+1)⁺** |
|---|---|---|---|---|
| Compound 3 | Ethyl-iodide | H | -CH₂CH₃ | 563 |
| Compound 4 | lodine-propane | H | -CH₂CH₂CH₃ | 577 |
| Compound 5 | Cyclopropyl-methylbromide | H | 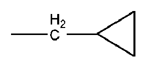 | 589 |
| Compound 6 | lodomethane | -CH₃ | -CH₃ | 563 |
| Compound 7 | Ethyl-iodide | -CH₂CH₃ | -CH₂CH₃ | 591 |
| Compound 8 | CH₃CH₂CH₂I | -CH₂CH₂CH₃ | -CH₂CH₂CH₃ | 619 |
| Compound 9 | CH₃CH₂I/CH₃I | -CH₃ | -CH₂CH₃ | 577 |
| Compound 10 | Allylbromide | H | 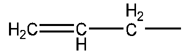 | 575 |
| Compound 11 | Propargylbromide | H | 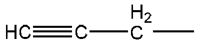 | 573 |
| Compound 12 | CH₃OBr | H | -OCH₃ | 565 |
| Compound 13 | CH₃OBr/CH₃l | -CH₃ | -OCH₃ | 579 |

### Embodiment 11: Preparation of (S)-N-(4-(3-fluorobenzyl-oxy)-3-chlorophenyl)-6-(5-(1-(amino)-2-(methylthio) ethyl)-thiazole-2-yl)quinazolin-4-amine

The process was the same as in embodiment 9, except that the raw material compound of embodiment 1 was replaced by the compound of embodiment 3. m/z(M+1)+: 552.

According to the preparation methods of embodiment 10, the compound in embodiment 10, as the starting material, was used to react with reagents to prepare the following compound:

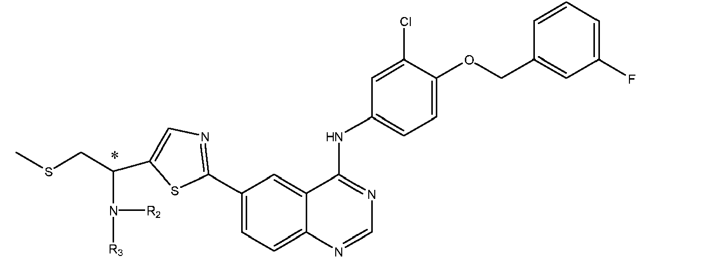

Remark: the carbon atom with * is (S)-configuration

| **Number** | **Reagent** | **R2 =** | **R3 =** | **m/z(M+1)⁺** |
|---|---|---|---|---|
| Compound 15 | CH₃I | H | -CH₃ | 566 |
| Compound 16 | CH₃CH₂-I | H | -CH₂CH₃ | 580 |
| Compound 17 | CH₃CH₂CH₂-I | H | -CH₂CH₂CH₃ | 594 |
| Compound 18 | Cyclopropyl-methylbromide | -H | 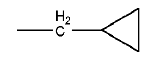 | 606 |
| Compound 19 | CH₃I | -CH₃ | -CH₃ | 580 |
| Compound 20 | CH₃CH₂I | -CH₂CH₃ | -CH₂CH₃ | 608 |
| Compound 21 | CH₃CH₂CH₂I | -CH₂CH₂CH₃ | -CH₂CH₂CH₃ | 636 |
| Compound 22 | CH₃CH₂I/CH₃I | -CH₃ | -CH₂CH₃ | 594 |
| Compound 23 | Allylbromide | H | 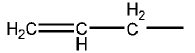 | 592 |
| Compound 24 | Propargylbromide | H | 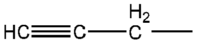 | 590 |
| Compound 25 | CH₃OBr | H | -OCH₃ | 582 |
| Compound 26 | CH₃OBr/CH₃I | -CH₃ | -OCH₃ | 596 |

### Embodiment 12: Preparation of (S)-N-(1-(3-fluorobenzyl)-1H-indazol-5-yl)-6-(5-(1-(amino) -2-(methylthio)ethyl)-furan-2-yl) quinazolin-4-amine

The process is as same as the process in embodiment 9, except that the raw material compound of embodiment 1 was replaced by compound of embodiment 5 to obtain the product (compound 27). m/z(M+1)+: 525.

According to the preparation methods of embodiment 10, the compound of embodiment 12, as a starting material, was used to react with reagents to prepare the following compound:

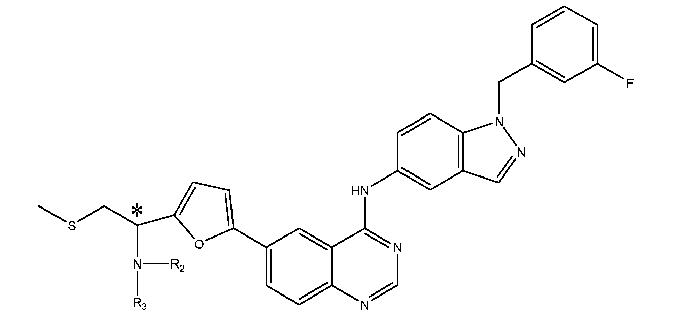

Remark: the carbon atom with * is (S)-configuration

| **Number** | **Reagent** | **R2 =** | **R3 =** | **m/z(M+1)⁺** |
|---|---|---|---|---|
| Compound 28 | CH₃I | H | -CH₃ | 539 |
| Compound 29 | CH₃CH₂-I | H | -CH₂CH₃ | 553 |
| Compound 30 | CH₃CH₂CH₂-I | H | -CH₂CH₂CH₃ | 567 |
| Compound 31 | Cyclopropyl-methylbromide | -H | 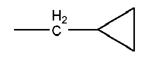 | 579 |
| Compound 32 | CH₃I | -CH₃ | -CH₃ | 553 |
| Compound 33 | CH₃CH₂I | -CH₂CH₃ | -CH₂CH₃ | 581 |
| Compound 34 | CH₃CH₂CH₂I | -CH₂CH₂CH₃ | -CH₂CH₂CH₃ | 609 |
| Compound 35 | CH₃CH₂I/CH₃I | -CH₃ | -CH₂CH₃ | 567 |
| Compound 36 | Allylbromide | H | 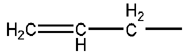 | 565 |
| Compound 37 | Propargylbromide | H | 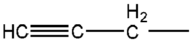 | 563 |
| Compound 38 | CH₃OBr | H | -OCH₃ | 555 |
| Compound 39 | CH₃OBr/CH₃I | -CH₃ | -OCH₃ | 569 |

### Embodiment 13: Preparation of (R)-N-(4-(3-fluorobenzyl-oxy)-3-chlorophenyl)-6-(5-(1-(amino)-2-(methylthio)ethyl)-furan-2-yl) quinazolin-4-amine

The process was the same as in embodiment 9, except that the raw material compound of embodiment 1 was replaced by compound of embodiment 2 to obtain the product designated compound 40. m/z(M+1)⁺: 535.

According to the preparation methods of embodiment 10, the compound obtained in embodiment 13, as the starting material, was used to react with reagents to prepare the following compound:

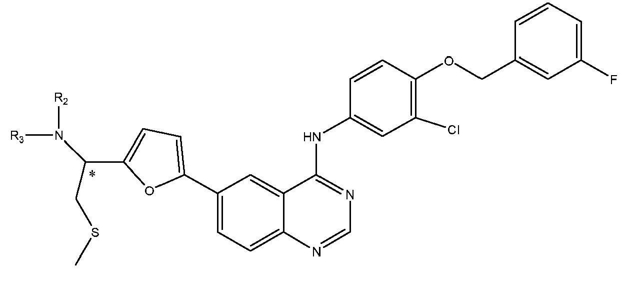

Remark: the carbon atom with * is (R)-configuration

| **Number** | **Reagent** | **R2 =** | **R3 =** | **m/z(M+1)⁺** |
|---|---|---|---|---|
| Compound 41 | lodomethane | H | -CH₃ | 549 |
| Compound 42 | Ethyl-iodide | H | -CH₂CH₃ | 563 |
| Compound 43 | lodine-propane | H | -CH₂CH₂CH₃ | 577 |
| Compound 44 | Cyclopropyl-methylbromide | H | 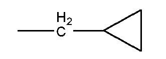 | 589 |
| Compound 45 | lodomethane | -CH₃ | -CH₃ | 563 |
| Compound 46 | Ethyl-iodide | -CH₂CH₃ | -CH₂CH₃ | 591 |
| Compound 47 | CH₃CH₂CH₂I | -CH₂CH₂CH₃ | -CH₂CH₂CH₃ | 619 |
| Compound 48 | CH₃CH₂l/CH₃I | -CH₃ | -CH₂CH₃ | 577 |
| Compound 49 | Allylbromide | H | 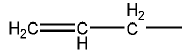 | 575 |
| Compound 50 | Propargylbromide | H | 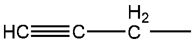 | 573 |
| Compound 51 | CH₃OBr | H | -OCH₃ | 565 |
| Compound 52 | CH₃OBr/CH₃I | -CH₃ | -OCH₃ | 579 |

### Embodiment 14: Preparation of (R)-N-(4-(3-fluorobenzyl-oxy)-3-chlorophenyl)-6-(5-(1-(amino)-2-(methylthio)ethyl)-thiazole-2-yl)quinazolin-4-amine

The process was the same as that in embodiment 9, except that the raw material compound of embodiment 1 was replaced by the compound of embodiment 4 to obtain the product (compound 53). m/z(M+1)+: 552.

According to the preparation methods in embodiment 10, in the compound in embodiment 14, as the starting material, was used to react with reagents to prepare the following compound:

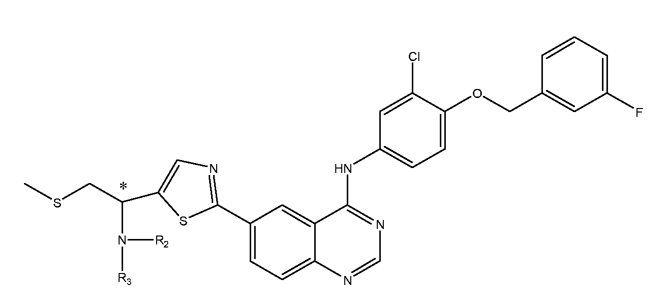

Remark: the carbon atom with * is (R)-configuration

| **Number** | **Reagent** | **R2 =** | **R3 =** | **m/z(M+1)⁺** |
|---|---|---|---|---|
| Compound 54 | CH₃I | H | -CH₃ | 566 |
| Compound 55 | CH₃CH₂-I | H | -CH₂CH₃ | 580 |
| Compound 56 | CH₃CH₂CH₂-I | H | -CH₂CH₂CH₃ | 594 |
| Compound 57 | Cyclopropyl-methylbromide | -H | 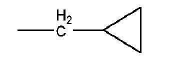 | 606 |
| Compound 58 | CH₃I | -CH₃ | -CH₃ | 580 |
| Compound 59 | CH₃CH₂I | -CH₂CH₃ | -CH₂CH₃ | 608 |
| Compound 60 | CH₃CH₂CH₂I | -CH₂CH₂CH₃ | -CH₂CH₂CH₃ | 636 |
| Compound 61 | CH₃CH₂I/CH₃I | -CH₃ | -CH₂CH₃ | 594 |
| Compound 62 | Allylbromide | H | 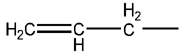 | 592 |
| Compound 63 | Propargylbromide | H | 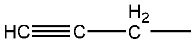 | 590 |
| Compound 64 | CH₃OBr | H | -OCH₃ | 582 |
| Compound 65 | CH₃OBr/CH₃I | -CH₃ | -OCH₃ | 596 |

### Embodiment 15: Preparation of (S)-N-(4-(3-fluorobenzyl-oxy)-3-chlorophenyl)-6-(5-(1-(amino)-2-(methylsulfinyl)ethyl)-furan-2-yl)quinazolin-4-amine

Under the protection of N₂, DMSO (0.4 mol) was dissolved in anhydrous THF (2000 ml) and cooled to -20°C. Then, n-BuLi (0.3 mol) was added drop-wisely to the solution and stirred for 10 min at the same temperature. The reaction solution was cooled below -80°C. Then, the solution of product of embodiment 1 (57.6 g, 0.1 mol) and anhydrous THF (200 ml) were added rapidly at -80°C and stirred for 10min. The reaction solution was decanted into saturated saline (3000 ml), and ethyl acetate (2000ml) was added to extract the product. The organic layer was washed with saturated saline (2000 ml), and dried with anhydrous magnesium sulfate. After filtration and concentration under reduced pressure, 42 g yellow solid were obtained.

The yellow solid obtained in the last step was dissolved in THF (1000 ml). The pH of the solution was adjusted to 1 with HCl-ethanol and stirred for 2 h at room temperature. To the solution, strong ammonia was added to adjust the pH to 9, and saturated saline (2000 ml) and ethyl acetate (1500 ml) were added to extract the product. The organic layer was dried with anhydrous magnesium sulfate. After filtration and concentration of the filtrate under reduced pressure, the remainder residue was purified by silica-gel column chromatography (eluent: ethyl acetate-ethyl acetate/THF = 5/1), and the fraction in need was collected and concentrated to obtain 20 g product (compound 66). m/z(M+1)+: 551.

### Embodiment 16: Preparation of (S)-N-(4-(3-fluorobenzyl-oxy)-3-chlorophenyl)-6-(5-(1-(methylamino)-2-(methylsulfinyl)ethyl)-furan-2-yl)quinazolin-4-amine

The process was the same as in embodiment 10, except that the raw material compound of embodiment 7 was replaced by compound of embodiment 15, and the product was designated as compound 67. m/z(M+1)+: 566.

According to the preparation methods in embodiment 10, the compound obtained in embodiment 15, as the starting material, was used to react with reagents to prepare the following compound:

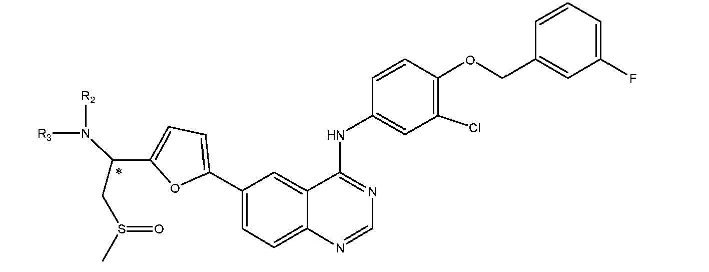

Remark: the carbon atom with * is (S)-configuration

| **Number** | **Reagent** | **R2 =** | **R3 =** | **m/z(M+1)⁺** |
|---|---|---|---|---|
| Compound 68 | Ethyl-iodide | H | -CH₂CH₃ | 579 |
| Compound 69 | lodine-propane | H | -CH₂CH₂CH₃ | 593 |
| Compound 70 | Cyclopropyl-methylbromide | H | 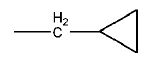 | 605 |
| Compound 71 | CH3I | -CH₃ | -CH₃ | 579 |
| Compound 72 | CH₃CH₂I | -CH₂CH₃ | -CH₂CH₃ | 607 |
| Compound 73 | CH₃CH₂CH₂I | -CH₂CH₂CH₃ | -CH₂CH₂CH₃ | 635 |
| Compound 74 | CH₃CH₂l/CH₃I | -CH₃ | -CH₂CH₃ | 593 |
| Compound 75 | Allylbromide | H | 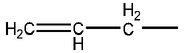 | 591 |
| Compound 76 | Propargylbromide | H | 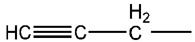 | 589 |
| Compound 77 | CH₃OBr | H | -OCH₃ | 581 |
| Compound 58 | CH₃OBr/CH₃I | -CH₃ | -OCH₃ | 595 |

### Embodiment 17: Preparation of (R)-N-(4-(3-fluorobenzyl-oxy)-3-chlorophenyl)-6-(5-(1-(amino)-2-(methylsulfinyl)ethyl)-furan-2-yl)quinazolin-4-amine

The process was the same in embodiment 15, except that the raw material compound of embodiment 1 was replaced by compound of embodiment 2, and the title compound was designated as compound 79. m/z(M+1)+: 551.

According to the preparation methods in embodiment 10, the compound obtained in embodiment 17, as the starting material, was used to react with reagents to prepare the following compound:

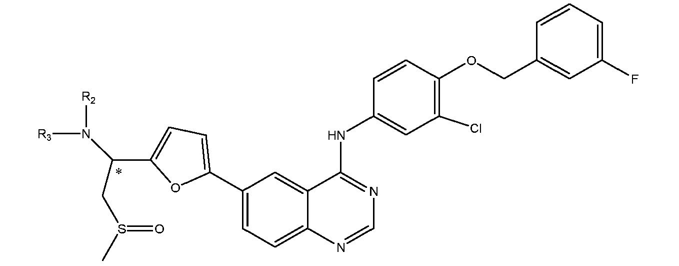

Remark: the carbon atom with * is (R)-configuration

| **Number** | **Reagent** | **R2 =** | **R3 =** | **m/z(M+1)⁺** |
|---|---|---|---|---|
| Compound 80 | CH₃I | H | -CH₃ | 565 |
| Compound 81 | CH₃CH₂-I | H | -CH₂CH₃ | 579 |
| Compound 82 | CH₃CH₂CH₂-I | H | -CH₂CH₂CH₃ | 593 |
| Compound 83 | Cyclopropyl-methylbromide | -H | 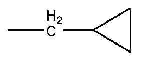 | 605 |
| Compound 84 | CH₃I | -CH₃ | -CH₃ | 579 |
| Compound 65 | CH₃CH₂I | -CH₂CH₃ | -CH₂CH₃ | 607 |
| Compound 86 | CH₃CH₂CH₂I | -CH₂CH₂CH₃ | -CH₂CH₂CH₃ | 635 |
| Compound 87 | CH₃CH₂I/CH₃I | -CH₃ | -CH₂CH₃ | 593 |
| Compound 88 | Allylbromide | H | 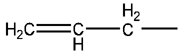 | 591 |
| Compound 89 | Propargylbromide | H | 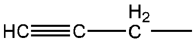 | 589 |
| Compound 90 | CH₃OBr | H | -OCH₃ | 581 |
| Compound 91 | CH₃OBr/CH₃I | -CH₃ | -OCH₃ | 595 |

### Embodiment 18: Preparation of (S)-N-(4-(3-fluorobenzyl-oxy)-3-chlorophenyl)-6-(5-(1-(amino)-2-(methylsulfonyl)ethyl)-furan-2-yl)quinazolin-4-amine

Method A: Under nitrogen protection, DMSO (37.6 g, 0.4 mol) was dissolved in anhydrous THF (2000 ml) and cooled to -20°C. Then, n-BuLi (0.3 mol) was added drop-wisely to the solution which was stirred for 30 min at the same temperature. The reaction solution was cooled below -80°C, and the solution of the product of embodiment 1 (57.6 g, 0.1 mol) and anhydrous THF (200ml) were added rapidly at -80°C. The solution was stirred for 10 min at the present temperature. The reaction solution was decanted into saturated saline (3000 ml), and ethyl acetate (2000 ml) was added to extract the mixture. The organic layer was washed with saturated saline (2000 ml) and dried with anhydrous magnesium sulfate. After filtration and concentration under reduced pressure, 42 g yellow solid were obtained.

The yellow solid obtained in the last step was dissolved in THF (1000 ml). The pH of the solution was adjusted to 1 with HCl-ethanol, and the solution was stirred for 2 h at room temperature. The pH of the solution was adjusted with strong ammonia to 9. Saturated saline (2000 ml) and ethyl acetate (1500 ml) were added to extract the mixture. The organic layer was dried with anhydrous magnesium sulfate. After filtration and concentration under reduced pressure, the residue from the organic layer was purified by silica-gel column chromatography (eluent: ethyl acetate-ethyl acetate/THF = 5/1). The fraction in need was collected and concentrated to obtain 20 g product designated as compound 92.

Method B: The products obtained in embodiment 9 (50 g) and embodiment 15 (50 g) and mixed solvent of methanol/water (7:3, 1000 ml) were added into a reaction flask. After all products were dissolved in the solvent, potassium peroxymonopersulfate (KHS05, 100 g) was added in batches under stirring for 2 h at room temperature. After filtration and the solid residue was washed with mixed solvent of methanol/water, and the pH of the combined filtrate was adjusted to 8 with saturated sodium bicarbonate solution. The solution was concentrated under reduced pressure, and then extracted with ethyl acetate (500 ml × 2). The organic layers were combined, and dried with anhydrous sodium sulfate. After filtration and concentration under reduced pressure, the residue was purified by silica-gel column chromatography (eluent: chloroform/methanol = 100:1) to obtain the product: 40 g, yellow solid. m/z(M+1)+: 567. e.e value: 95.2% [(S): 97.6%, (R): 2.4%].

According to the preparation methods in embodiment 10, the compound obtained in embodiment 18, as the starting material, was used to react with reagents to prepare the following compound:

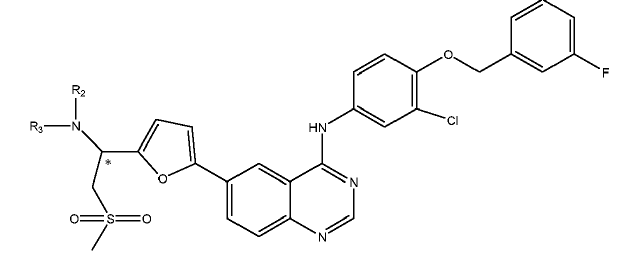

Remark: the carbon atom with * is (S)-configuration

| **Number** | **Reagent** | **R2 =** | **R3 =** | **m/z(M+1)⁺** | **e.e value%** |
|---|---|---|---|---|---|
| Compound 93 | CH₃I | H | -CH₃ | 581 | 95.2 |
| Compound 94 | CH₃CH₂I | H | -CH₂CH₃ | 595 | 95.3 |
| Compound 95 | CH₃CH₂CH₂I | H | -CH₂CH₂CH₃ | 609 | 95.3 |
| Compound 96 | Cyclopropyl-methylbromide | H | 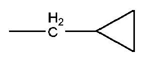 | 621 | 95.2 |
| Compound 97 | CH₃I | -CH₃ | -CH₃ | 595 | 95.2 |
| Compound 98 | CH₃CH₂I | -CH₂CH₃ | -CH₂CH₃ | 623 | 95.2 |
| Compound 99 | CH₃CH₂CH₂I | -CH₂CH₂CH₃ | -CH₂CH₂CH₃ | 651 | 95.2 |
| Compound 100 | CH₃CH₂I/CH₃I | -CH₃ | -CH₂CH₃ | 609 | 95.4 |
| Compound 101 | Allylbromide | H | 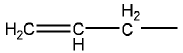 | 607 | 95.2 |
| Compound 102 | Propargyl-bromide | H | 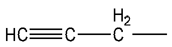 | 605 | 95.2 |
| Compound 103 | CH₃OBr | H | -OCH₃ | 597 | 95.1 |
| Compound 104 | CH₃OBr/CH₃I | -CH₃ | -OCH₃ | 611 | 95.2 |

### Embodiment 19: Preparation of (R)-N-(4-(3-fluorobenzyl-oxy)-3-chlorophenyl)-6-(5-(1-(amino)-2-(methylsulfonyl)ethyl)-furan-2-yl)quinazolin-4-amine

Method A: The process was the same as in method A of embodiment 18, except that the raw material compound of embodiment 1 was replaced by the compound of embodiment 2. The product was designated as compound 105. m/z(M+1)+: 567. e.e value: 95.8% [(R): 97.9%, (S): 2.1%].

Method B: The process was the same as in method B of embodiment 18, except that the raw material compounds of embodiment 9 and embodiment 15 were replaced by the compound of embodiment 13 or embodiment 17.

According to the preparation methods of embodiment 10, the compound obtained in embodiment 19, as the starting material, was used to react with reagents to prepare the following compounds:

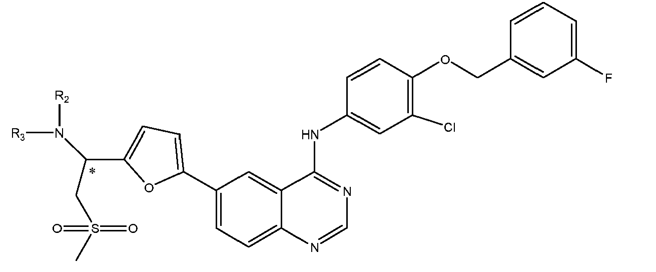

Remark: the carbon atom with * is (R)-configuration

| **Number** | **Reagent** | **R2 =** | **R3 =** | **m/z(M+1)⁺** | **e.e value%** |
|---|---|---|---|---|---|
| Compound 106 | CH₃I | H | -CH₃ | 581 | 95.9 |
| Compound 107 | CH₃CH₂I | H | -CH₂CH₃ | 595 | 95.8 |
| Compound 108 | CH₃CH₂CH₂I | H | -CH₂CH₂CH₃ | 609 | 95.8 |
| Compound 109 | Cyclopropyl-methylbromide | H | 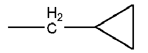 | 621 | 95.9 |
| Compound 110 | CH₃I | -CH₃ | -CH₃ | 595 | 96.0 |
| Compound 111 | CH₃CH₂I | -CH₂CH₃ | -CH₂CH₃ | 623 | 95.8 |
| Compound 112 | CH₃CH₂CH₂I | -CH₂CH₂CH₃ | -CH₂CH₂CH₃ | 651 | 95.8 |
| Compound 113 | CH₃CH₂I/CH₃I | -CH₃ | -CH₂CH₃ | 609 | 95.7 |
| Compound 114 | Allylbromide | H | 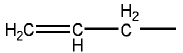 | 607 | 95.8 |
| Compound 115 | Propargyl-bromide | H | 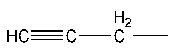 | 605 | 95.9 |
| Compound 116 | CH₃OBr | H | -OCH₃ | 597 | 95.8 |
| Compound 117 | CH₃OBr/CH₃I | -CH₃ | -OCH₃ | 611 | 95.8 |

### Embodiment 20: Preparation of (S)-N-(4-(3-fluorobenzyl-oxy)-3-chlorophenyl)-6-(5-(1-(amino)-2-(methylsulfonyl)ethyl)-thiazole-2-yl)quinazolin-4-amine

The process was the same as in method A of embodiment 18, except that the raw material compound of embodiment 1 was replaced by the compound of embodiment 3. The product was designated as compound 118. m/z(M+1)+: 584. e.e value: 91.4% [(S): 95.7%, (R): 4.3%].

According to the preparation methods of embodiment 10, the compound obtained in embodiment 20, as the starting material, was used to react with reagents to prepare the following compounds:

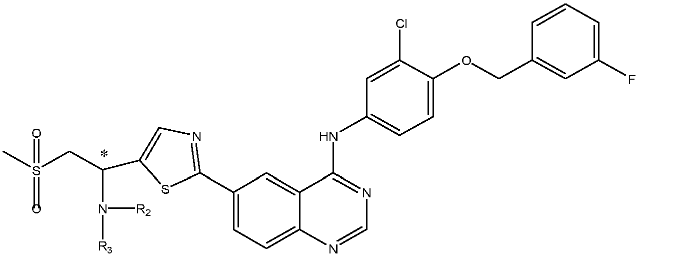

Remark: the carbon atom with * is (S)-configuration

| **Number** | **Reagent** | **R2 =** | **R3 =** | **m/z(M+1)⁺** | **e.e value%** |
|---|---|---|---|---|---|
| Compound 119 | CH₃I | H | -CH₃ | 598 | 91.5 |
| Compound 120 | CH₃CH₂I | H | -CH₂CH₃ | 612 | 91.7 |
| Compound 121 | CH₃CH₂CH₂I | H | -CH₂CH₂CH₃ | 626 | 91.6 |
| Compound 122 | Cyclopropyl-methylbromide | H | 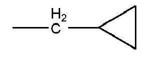 | 638 | 91.5 |
| Compound 123 | CH₃I | -CH₃ | -CH₃ | 612 | 91.4 |
| Compound 124 | CH₃CH₂I | -CH₂CH₃ | -CH₂CH₃ | 640 | 91.8 |
| Compound 125 | CH₃CH₂CH₂I | -CH₂CH₂CH₃ | -CH₂CH₂CH₃ | 668 | 91.5 |
| Compound 126 | CH₃CH₂I/CH₃I | -CH₃ | -CH₂CH₃ | 626 | 91.5 |
| Compound 127 | Allylbromide | H | 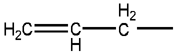 | 624 | 91.6 |
| Compound 128 | Propargyl-bromide | H | 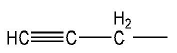 | 622 | 91.7 |
| Compound 129 | CH₃OBr | H | -OCH₃ | 614 | 91.4 |
| Compound 130 | CH₃OBr/CH₃I | -CH₃ | -OCH₃ | 628 | 91.2 |

### Embodiment 21: Preparation of (R)-N-(4-(3-fluorobenzyl-oxy)-3-chlorophenyl)-6-(5-(1-(amino)-2-(methylsulfonyl)ethyl)-thiazole-2-yl)quinazolin-4-amine

The process was the same as in method A of embodiment 18, except that the raw material compound of embodiment 1 was replaced by compound of embodiment 4. The product was designated as compound 131. m/z(M+1)+: 584. e.e value: 92.2% [(R): 96.1%, (S): 3.9%].

According to the preparation method in embodiment 10, the compound obtained in embodiment 21, as the starting material, was used to react with reagents to prepare the following compound:

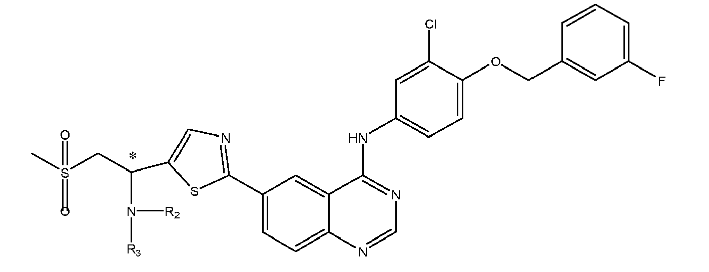

Remark: the carbon atom with * is (R)-configuration

| **Number** | **Reagent** | **R2 =** | **R3 =** | **m/z(M+1)⁺** | **e.e value%** |
|---|---|---|---|---|---|
| Compound 132 | CH₃I | H | -CH₃ | 598 | 92.3 |
| Compound 133 | CH₃CH₂I | H | -CH₂CH₃ | 612 | 92.0 |
| Compound 134 | CH₃CH₂CH₂I | H | -CH₂CH₂CH₃ | 626 | 92.2 |
| Compound 135 | Cyclopropyl-methylbromide | H | 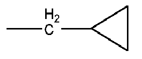 | 638 | 92.1 |
| Compound 136 | CH₃I | -CH₃ | -CH₃ | 612 | 92.2 |
| Compound 137 | CH₃CH₂I | -CH₂CH₃ | -CH₂CH₃ | 640 | 92.3 |
| Compound 138 | CH₃CH₂CH₂I | -CH₂CH₂CH₃ | -CH₂CH₂CH₃ | 668 | 92.0 |
| Compound 139 | CH₃CH₂I/CH₃I | -CH₃ | -CH₂CH₃ | 626 | 92.4 |
| Compound 140 | Allylbromide | H | 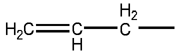 | 624 | 92.2 |
| Compound 141 | Propargyl-bromide | H | 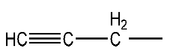 | 622 | 92.2 |
| Compound 142 | CH₃OBr | H | -OCH₃ | 614 | 92.1 |
| Compound 143 | CH₃OBr/CH₃I | -CH₃ | -OCH₃ | 628 | 92.3 |

### Embodiment 22: Preparation of (S)-N-(1-(3-fluorobenzyl)-1H-indazol-5-yl)-6-(5-(1-(amino) -2-(methylsulfonyl)ethyl)-furan-2-yl)quinazolin-4-amine

Method A: The process was the same as in method A of embodiment 18, except that the raw material compound of embodiment 1 was replaced by the compound of embodiment 5. The product was designated as compound 144. m/z(M+1)+: 557. e.e value: 93.0% [(S): 96.5%, (R): 3.5%].

Method B: The process was the same as in method B of embodiment 18, except that the raw material compound of embodiment 15 was replaced by the compound of embodiment 12.

According to the preparation methods in embodiment 10, the compound obtained in embodiment 22, as the starting material, was used to react with reagents to prepare the following compound:

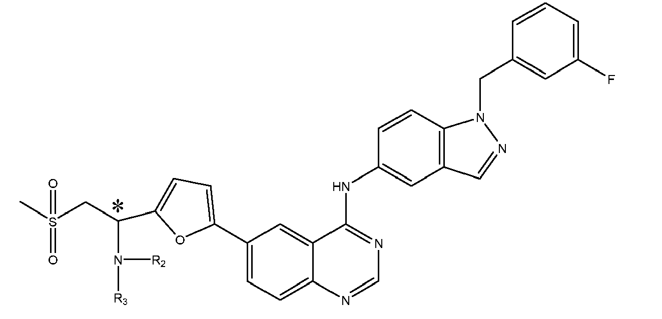

Remark: the carbon atom with * is (S)-configuration

| **Number** | **Reagent** | **R2 =** | **R3 =** | **m/z(M+1)⁺** | **e.e value%** |
|---|---|---|---|---|---|
| Compound 145 | CH₃I | H | -CH₃ | 571 | 93.1 |
| Compound 146 | CH₃CH₂I | H | -CH₂CH₃ | 585 | 93.0 |
| Compound 147 | CH₃CH₂CH₂I | H | -CH₂CH₂CH₃ | 599 | 93.2 |
| Compound 148 | Cyclopropyl-methylbromide | H | 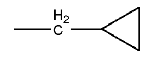 | 611 | 93.0 |
| Compound 149 | CH₃I | -CH₃ | -CH₃ | 585 | 93.1 |
| Compound 150 | CH₃CH₂I | -CH₂CH₃ | -CH₂CH₃ | 613 | 93.1 |
| Compound 151 | CH₃CH₂CH₂I | -CH₂CH₂CH₃ | -CH₂CH₂CH₃ | 641 | 93.2 |
| Compound 152 | CH₃CH₂I/CH₃I | -CH₃ | -CH₂CH₃ | 599 | 93.0 |
| Compound 153 | Allylbromide | H | 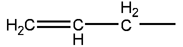 | 597 | 93.0 |
| Compound 154 | Propargyl-bromide | H | 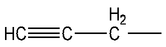 | 595 | 93.0 |
| Compound 155 | CH₃OBr | H | -OCH₃ | 587 | 93.1 |
| Compound 156 | CH₃OBr/CH₃I | -CH₃ | -OCH₃ | 601 | 93.0 |

### TEST 1: Evaluation of antitumor activity in vitro Experiment method: SRB Cell strains: A431; MCF-7

Experiment design: Cells were incubated with different concentrations of compounds for 72 h. The inhibition of cell proliferation by the compounds was evaluated by SRB, followed by calculation of the inhibition rate. Then value of IC₅₀ was calculated by using -logit method based on the inhibition rate and used to compare *in vitro* antitumor activity of the compounds. Method for calculating the inhibition rate: Inhibition rate (%) = (OD value of control group - OD value of drug group)/OD value of control group × 100%.

Experiment results:

| **Number** | **IC₅₀(nm)** | |
|---|---|---|
| | **A431(72h)** | **MCF-7(72h)** |
| Lapatinib (positive control) | 643.1 | 8468 |
| Compound 92 | 422 | 9336 |
| Compound 93 | 459.6 | 8861 |
| Compound 94 | 652.5 | 7853 |
| Compound 95 | 452.8 | 7900 |
| Compound 96 | 578.4 | 6700 |
| Compound 97 | 734.1 | 6666 |
| Compound 98 | 677.2 | 7658 |
| Compound 99 | 758.8 | 5922 |
| Compound 100 | 488.6 | 9896 |
| Compound 101 | 588.3 | 10426 |
| Compound 102 | 456.9 | 8900 |
| Compound 103 | 478.8 | 8824 |
| Compound 104 | 632.2 | 9004 |
| Compound 105 | 663.7 | 7865 |
| Compound 106 | 469.1 | 7800 |
| Compound 107 | 578.4 | 6766 |
| Compound 108 | 528.9 | 5900 |
| Compound 109 | 755.8 | 6211 |
| Compound 110 | 767.7 | 5669 |
| Compound 111 | 699.4 | 6823 |
| Compound 112 | 855.3 | 6218 |
| Compound 113 | 758.2 | 7066 |
| Compound 114 | 488.9 | 7900 |
| Compound 115 | 535.7 | 7580 |
| Compound 116 | 440.7 | 4800 |
| Compound 117 | 635.5 | 7626 |
| Compound 118 | 524.6 | 6687 |
| Compound 119 | 587.8 | 6121 |
| Compound 120 | 658.1 | 7562 |
| Compound 121 | 465.0 | 6387 |
| Compound 122 | 399.8 | 5968 |
| Compound 123 | 498.8 | 6328 |
| Compound 124 | 684.7 | 7980 |
| Compound 125 | 523.0 | 9800 |
| Compound 126 | 487.9 | 7764 |
| Compound 127 | 521.4 | 5889 |
| Compound 128 | 672.2 | 6336 |
| Compound 129 | 666.8 | 9711 |
| Compound 130 | 536.3 | 8588 |
| Compound 131 | 582.1 | 8525 |
| Compound 132 | 388.7 | 9907 |
| Compound 133 | 554.4 | 7887 |
| Compound 134 | 579.5 | 6510 |
| Compound 135 | 488.6 | 6190 |
| Compound 136 | 448.7 | 5430 |
| Compound 137 | 589.2 | 5470 |
| Compound 138 | 426.3 | 4599 |
| Compound 139 | 452.4 | 4130 |
| Compound 140 | 647.7 | 4020 |
| Compound 141 | 485.9 | 7624 |
| Compound 142 | 499.5 | 7865 |
| Compound 143 | 788.2 | 5090 |
| Compound 144 | 710.6 | 4970 |
| Compound 145 | 581.3 | 8240 |
| Compound 146 | 489.1 | 5460 |
| Compound 147 | 405.7 | 8010 |
| Compound 148 | 442.3 | 7001 |
| Compound 149 | 348.9 | 7230 |
| Compound 150 | 624.1 | 6674 |
| Compound 151 | 637.2 | 6080 |
| Compound 152 | 600.8 | 6540 |
| Compound 153 | 857.2 | 5870 |
| Compound 154 | 624.3 | 4897 |
| Compound 155 | 479.6 | 8588 |
| Compound 156 | 427.6 | 5520 |

### TEST 2: Effect of compound 97(±), compound 97, compound 110 and lapatinib (positive control) on xenografts in nude mice with lung cancer Calu-3 Remark: "±" means that the compound is racemic.

### 1. Abstract

Evaluation and comparison of effects of compound 97 (±), compound 97, compound 110, and lapatinib (positive control) on xenografts in nude mice with lung cancer Calu-3. Compound 97, compound 110 and lapatinib all inhibited the growth of human lung cancer Calu-3 significantly. The descending order of efficiency is compound 110, compound 97 (±), compound 97, Lapatinib. Compound 110 can cause most tumors in mice to shrink. Mice can well tolerate all the compounds mentioned above.

### 2. Purpose of this experiment

The purpose of the experiment is the evaluation and comparison of the effects of compound 97 (±), compound 97, compound 110, and lapatinib on xenografts in nude mice with lung cancer Calu-3.

### 3. Animals for this experiment

BALB/cA-nude mice, 6-7 weeks, ♀, purchased from ShangHai slac laboratory animal Co., Ltd.; Certificate NO.: SCXK ( 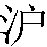 ) 2007-0005. Feeding condition: SPF grade.

### 4. Experimental procedures

Calu-3 cells were injected subcutaneously into nude mice. Animals were divided into groups (d0) randomly after tumors have grown to 150-300 mm³. Dosage and dosage regimen design can be seen in Table 1. Tumor volume was measured 2-3 times per week. Mice were weighed and data were recorded. Calculation formula for tumor volume (V) is as below: V= 1/2 × a × b², wherein, a and b represents length and width, respectively.

### 5. Experimental results

Compound 97, compound 110 and lapatinib all inhibited the growth of human lung cancer Calu-3 significantly. The sort descending order of efficiency is: compound 110, compound 97 (±), compound 97, and lapatinib. Compound 110 can cause 5/6 of the tumors in mice to shrink, with tumor in one mouse shrinking more than 50% in volume. Mice can well tolerate all compounds mentioned above.

**Table 1: Effect of compound 97 (±), compound 97, compound 110, and lapatinib on xenografts in nude mice with lung cancer Calu-3.**

| **Group** | **Drug Cycle** | **Administration route** | **Initial tumor volume(mm³)** | | **Final tumor volume (mm³)** | | **Relative tumor volume** | | **% T/C** | **Final tumor inhibition rate (%)** | **Relative value (final vs. control)** | **The recession/ group** | **Mouse number** |
|---|---|---|---|---|---|---|---|---|---|---|---|---|---|
| | | | **D0** | **SEM** | **D16** | **SEM** | **D16** | **SEM** | **D16** | **D16** | **D16** | | |
| Vehicle | QD×17 | PO | 268.0 | ±19.3 | 887.7 | ±109.3 | 3.4 | ±0.4 | 100 | 0 | _ | 0 | 10 |
| Compound 97(±) 146.5mg/kg | QD×17 | PO | 255.2 | ±28.8 | 279.6 | ±30.9 | 1.1 | ±0.1 | 33 | 67 | 0.001 | 0 | 6 |
| Compound 97(+) 146.6mg/kg | QD×17 | PO | 291.2 | ±14.8 | 525.4 | ±104.1 | 1.9 | ±0.5 | 56 | 44 | 0.037 | 0 | 6 |
| Compound 110(-) 146.7mg/kg | QD×17 | PO | 249.1 | ±19.3 | 187.4 | ±24.4 | 0.8 | ±0.1 | 23 | 77 | 0.000 | 1 | 6 |
| Lapatinib 146.7mg/kg | QD×17 | PO | 262.3 | ±21.6 | 526.5 | ±98.1 | 2.0 | ±0.3 | 58 | 42 | 0.027 | 0 | 6 |

| | | | | | | | | | | | | | |
|---|---|---|---|---|---|---|---|---|---|---|---|---|---|
| D0: time of the first administration; D16: the 17^{th} day after administration; n = 10 for control group, n = 6 for drug group; T represents mean tumor volume of drug group; C represents mean tumor volume of control group. | | | | | | | | | | | | | |

## Claims

1. Compounds of general formula (I) and pharmaceutically acceptable salts thereof, 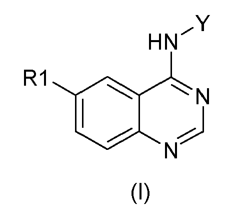 wherein R1 represents 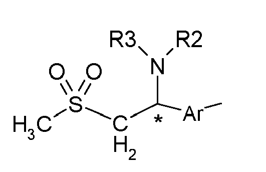 wherein Ar is selected from substituted or unsubstituted furan or thiazole, said substituents being selected from halogen atoms, C₁₋₄ alkyl or C₁₋₄ alkoxy, and the number of the substituents being 1 or 2;
the carbon atom with * is a chiral carbon atom; and
R2 and R3 are independently selected from (1) hydrogen, (2) alkyl, (3) alkenyl, (4) alkynyl, (5) alkoxy, (6) alkoxy alkyl, (7) cycloalkyl, or (8) cycloalkyl alkyl;
Y is selected from phenyl or 1H-indazolyl, which is optionally substituted with R4, R5; wherein R4 is selected from benzyl, halo-, dihalo- or trihalo-benzyl, benzyloxy, halo-, dihalo- or trihalo-benzyloxy; and R5 is selected from hydrogen, hydroxy, halogen atoms, C₁₋₄ alkyl, C₁₋₄ alkoxy, amino, cyano or trifluoromethyl.

2. The compounds of claim 1, wherein Ar is selected from unsubstituted furan or thiazole.

3. The compounds of claim 1 or 2, wherein R2 and R3 are independently selected from: (1) hydrogen, (2) C₁-₄ alkyl, (3) C₂-₅ alkenyl, (4) C₁-₄ alkoxy, (5) C₁-₄ alkoxy C₁-₄ alkyl, (6) C₃₋₈ cycloalkyl, or (7) C₃₋₈ cycloalkyl-C₁₋₄ alkyl.

4. The compounds of any one of claims 1 to 3, wherein R4 is selected from benzyl, halo-benzyl, halo-benzyloxy, preferably halo-benzyl or halo-benzyloxy; and R5 is selected from hydrogen, halogen atoms, C¹⁻⁴ alkyl or C¹⁻⁴ alkoxy.

5. The compounds of any one of claims 1 to 4, wherein carbon atom with * exists in the form of a single enantiomer of (R) or (S).

6. The compounds of any one of claims 1 to 4, wherein carbon atom with * exists in the form of rich in enantiomer of (R) or (S), preferably the percentage of enantiomer of (R) or (S) is ≥ 90%.

7. The compounds of any one of claims 1 to 4, which comprise:
(R)-N-(4-(3-fluorobenzyl-oxy)-3-chlorophenyl)-6-(5-(1-(amino)-2-(methylsulfonyl) ethyl)-furan-2-yl) quinazolin-4-amine;
(S)-N-(4-(3-fluorobenzyl-oxy)-3-chlorophenyl)-6-(5-(1-(amino)-2-(methylsulfonyl) ethyl)-furan-2-yl) quinazolin-4-amine;
(R)-N-(4-(3-fluorobenzyl-oxy)-3-chlorophenyl)-6-(5-(1-(methylamino)-2-(methylsulfonyl)ethyl)-furan-2-yl) quinazolin-4-amine;
(R)-N-(4-(3-fluorobenzyl-oxy)-3-chlorophenyl)-6-(5-(1-(ethylamino)-2-(methylsulfonyl)ethyl)-furan-2-yl) quinazolin-4-amine;
(R)-N-(4-(3-fluorobenzyl-oxy)-3-chlorophenyl)-6-(5-(1-(propyl-amino)-2-(methylsulfonyl)ethyl)-furan-2-yl) quinazolin-4-amine;
(R)-N-(4-(3-fluorobenzyl-oxy)-3-chlorophenyl)-6-(5-(1-(cyclopropyl-methylamino) -2-(methylsulfonyl)ethyl)-furan-2-yl) quinazolin-4-amine;
(R)-N-(4-(3-fluorobenzyl-oxy)-3-chlorophenyl)-6-(5-(1-(N,N-dimethyl-amino)-2-(methylsulfonyl)ethyl)-furan-2-yl) quinazolin-4-amine;
(R)-N-(4-(3-fluorobenzyl-oxy)-3-chlorophenyl)-6-(5-(1-(N,N-diethyl-amino)-2-(methylsulfonyl)ethyl)-furan-2-yl) quinazolin-4-amine;
(R)-N-(4-(3-fluorobenzyl-oxy)-3-chlorophenyl)-6-(5-(1-(N,N-dipropyl-amino)-2-(methylsulfonyl)ethyl)-furan-2-yl) quinazolin-4-amine;
(R)-N-(4-(3-fluorobenzyloxy)-3-chlorophenyl)-6-(5-(1-(N-methyl, N-ethyl-amino)-2-(methylsulfonyl)ethyl)-furan-2-yl) quinazolin-4-amine;
(R)-N-(4-(3-fluorobenzyl-oxy)-3-chlorophenyl)-6-(5-(1-(allyl-amino)-2-(methylsulfonyl)ethyl)-furan-2-yl) quinazolin-4-amine;
(R)-N-(4-(3-fluorobenzyl-oxy)-3-chlorophenyl)-6-(5-(1-(propargyl-amino)-2-(methyl-sulfonyl)ethyl)-furan-2-yl) quinazolin-4-amine;
(S)-N-(4-(3-fluorobenzyl-oxy)-3-chlorphenyl)-6-(5-(1-(methyl-amino)-2-(methylsulfonyl)ethyl)-furan-2-yl)quinazolin-4-amine;
(S)-N-(4-(3-fluorobenzyl-oxy)-3-chlorophenyl)-6-(5-(1-(ethyl-amino)-2-(methylsulfonyl)ethyl)-furan-2-yl) quinazolin-4-amine;
(S)-N-(4-(3-fluorobenzyl-oxy)-3-chlorophenyl)-6-(5-(1-(propyl-amino)-2-(methylsulfonyl)ethyl)-furan-2-yl) quinazolin-4-amine;
(S)-N-(4-(3-fluorobenzyl-oxy)-3-chlorophenyl)-6-(5-(1-(cyclopropyl-methylamino) -2-(methylsulfonyl)ethyl)-furan-2-yl) quinazolin-4-amine;
(S)-N-(4-(3-fluorobenzyl-oxy)-3-chlorophenyl)-6-(5-(1-(N,N-dimethyl-amino)-2-(methylsulfonyl)ethyl)-furan-2-yl) quinazolin-4-amine;
(S)-N-(4-(3-fluorobenzyl-oxy)-3-chlorophenyl)-6-(5-(1-(N,N-diethyl-amino)-2-(methylsulfonyl)ethyl)-furan-2-yl) quinazolin-4-amine;
(S)-N-(4-(3-fluorobenzyl-oxy)-3-chlorophenyl)-6-(5-(1-(N,N-dipropyl-amino)-2-(methylsulfonyl)ethyl)-furan-2-yl) quinazolin-4-amine;
(S)-N-(4-(3-fluorobenzyl-oxy)-3-chlorophenyl)-6-(5-(1-(N-methyl, N-ethyl-amino)-2-(methylsulfonyl)ethyl)-furan-2-yl) quinazolin-4-amine;
(S)-N-(4-(3-fluorobenzyl-oxy)-3-chlorophenyl)-6-(5-(1-(allyl-amino)-2-(methylsulfonyl) ethyl)-furan-2-yl) quinazolin-4-amine;
(S)-N-(4-(3-fluorobenzyl-oxy)-3-chlorophenyl)-6-(5-(1-(propargyl-amino)-2-(methylsulfonyl)ethyl)-furan-2-yl) quinazolin-4-amine;
(S)-N-(1-(3-fluorobenzyl)-1H-indazol-5-yl)-6-(5-(1-(amino)-2-(methylsulfonyl) ethyl)-furan-2-yl) quinazolin-4-amine;
(S)-N-(1-(3-fluorobenzyl)-1H-indazol-5-yl)-6-(5-(1-(methylamino)-2-(methylsulfonyl) ethyl)-furan-2-yl) quinazolin-4-amine;
(S)-N-(1-(3-fluorobenzyl)-1H-indazol-5-yl)-6-(5-(1-(ethyl-amino)-2-(methylsulfonyl) ethyl)-furan-2-yl) quinazolin-4-amine;
(S)-N-(1-(3-fluorobenzyl)-1H-indazol-5-yl)-6-(5-(1-(propyl-amino)-2-(methylsulfonyl) ethyl)-furan-2-yl) quinazolin-4-amine;
(S)-N-(1-(3-fluorobenzyl)-1H-indazol-5-yl)-6-(5-(1-(N,N-dimethyl-amino)-2-(methylsulfonyl)ethyl)-furan-2-yl) quinazolin-4-amine;
(S)-N-(1-(3-fluorobenzyl)-1H-indazol-5-yl)-6-(5-(1-(cyclopropyl-methyl-amino)-2-(methylsulfonyl)ethyl)-furan-2-yl) quinazolin-4-amine;
(S)-N-(1-(3-fluorobenzyl)-1H-indazol-5-yl)-6-(5-(1-(allyl-amino)-2-(methylsulfonyl) ethyl)-furan-2-yl) quinazolin-4-amine; and
(S)-N-(1-(3-fluorobenzyl)-1H-indazol-5-yl)-6-(5-(1-(propargyl-amino)-2-(methylsulfonyl)ethyl)-furan-2-yl) quinazolin-4-amine.

8. Method for the preparation of compounds of general formula (I) as defined in any one of claims 1 to 7, comprising:
(1) reacting the compounds of general formula (II) with tert-butylsulfinamide to prepare the compounds of general formula (III); 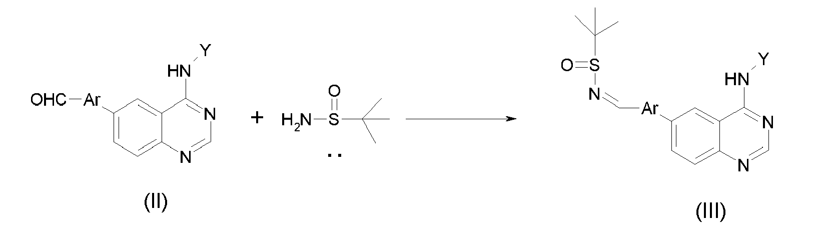
(2) reacting the compounds of general formula (III) with the compounds of general formula(IV) to prepare the compounds of general formula (V); 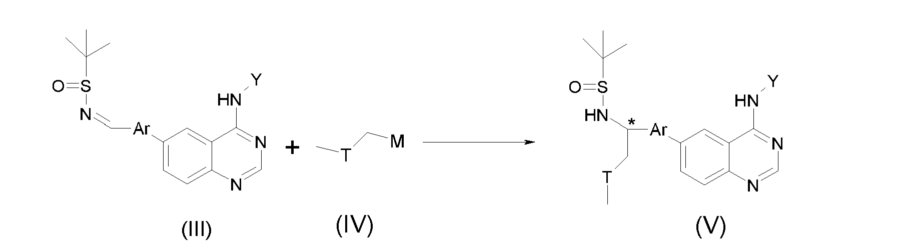
(3) generating the compounds of general formula (VI) from the compounds of general formula (V) in acid condition; 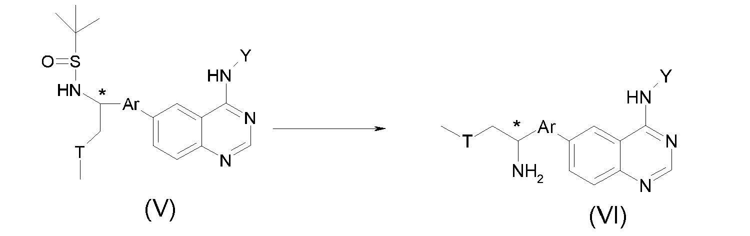
(4) reacting the compounds of general formula (VI) with reagent R2-L or R3-L to prepare the compounds of general formula (VII); and 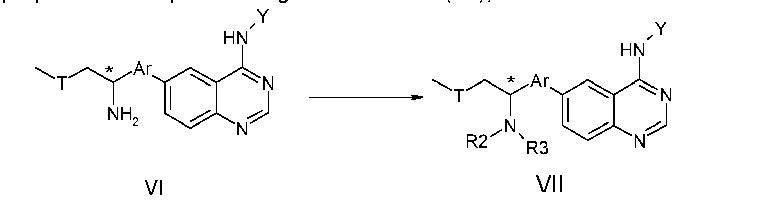
(5) reacting the compounds of general formula (VII) with oxidant to prepare the compound of general formula (I); 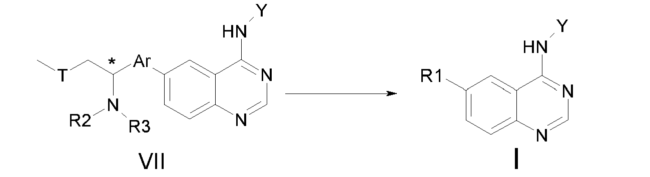 wherein R1, Y, Ar, R2, R3, and carbon atom with * are as defined in claim 1;
T is sulfur atom or sulfinyl;
tert-butylsulfinamide is optically pure, and exists in the form of a single enantiomer or rich in an enantiomer of (R) or (S);
M is alkali metal ion or halo-alkaline earth metal ions, selected from Li⁺, Na ⁺, K ⁺, [MgCl] ⁺ or [MgBr]⁺; and
L is leaving group, selected from halogen atoms or sulfonyloxyl group.

9. The method of claim 8, wherein the acid in step (3) is selected from hydrochloric acid, sulfuric acid, nitric acid, phosphoric acid, trifluoroacetic acid or the mixture of the foregoing acids, and preferably is hydrochloric acid; and the oxidant in step (5) is selected from chloroperoxybenzoic acid, peracetic acid, hydrogen peroxide or potassium monopersulfate, preferably potassium monopersulfate.

10. Method for the preparation of compounds of general formula (I) as defined in any one of claims 1 to 7, comprising:
(1) reacting the compounds of general formula (II) with tert-Butylsulfinamide to prepare the compound of general formula (III); 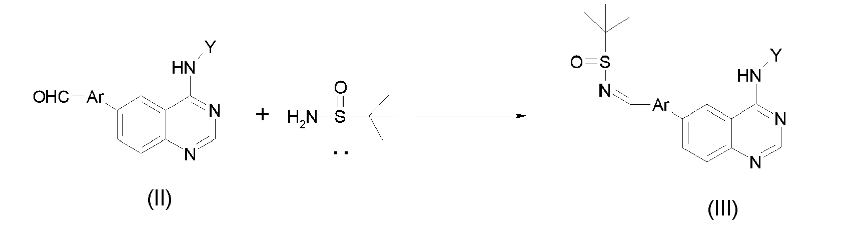
(2) reacting the compounds of general formula (III) with the compounds of general formula (A) to prepare the compound of general formula (B); 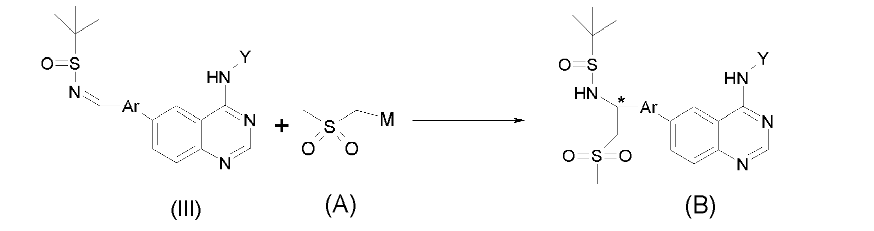
(3) generating the compounds of general formula (C) from the compounds of general formula (B) in acidic condition; and 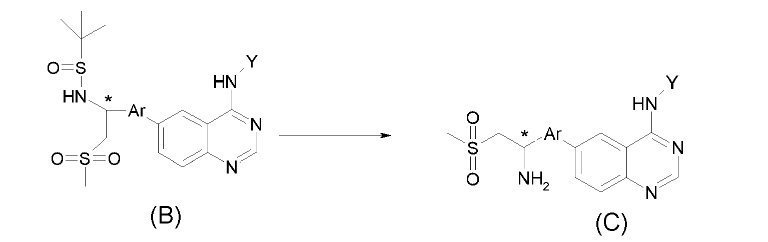
(4) reacting the compounds of general formula (C) with reagent R2-L or R3-L to prepare the compounds of general formula (I); 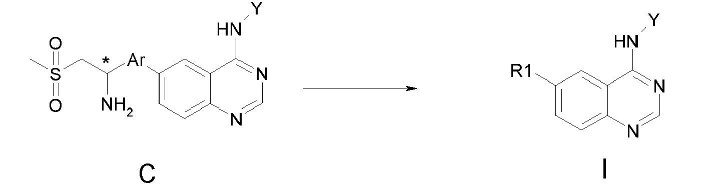 wherein R1, Y, Ar, R2, R3, and the carbon atom with * are as defined in claim 1; Tert-Butylsulfinamide, M, L are as defined in claim 11; and the acid in step (3) is selected from hydrochloric acid, sulfuric acid, nitric acid, phosphoric acid, trifluoroacetic acid or the mixture of the foregoing acids, and preferably is hydrochloric acid.

11. Compounds of general formula (VII), which are the intermediate compounds for synthesis of the compounds of general formula (I) in claim 1: 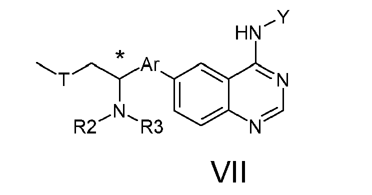 wherein Y, Ar, R2, R3, and the carbon atom with * are as defined in claim 1; and T is a sulfur atom or sulfinyl.

12. The compounds of claim 11, which comprise:
(R)-N-(4-(3-fluorobenzy-oxy)-3-chlorophenyl)-6-(5-(1-(amino)-2-(methylthio)ethyl)-furan-2-yl) quinazolin-4-amine;
(R)-N-(4-(3-fluorobenzyl-oxy)-3-chlorophenyl)-6-(5-(1-(methyl-amino)-2-(methylthio) ethyl)-furan-2-yl) quinazolin-4-amine;
(R)-N-(4-(3-fluorobenzyl-oxy)-3-chlorophenyl)-6-(5-(1-(ethyl-amino)-2-(methylthio) ethyl)-furan-2-yl) quinazolin-4-amine;
(R)-N-(4-(3-fluorobenzyl-oxy)-3-chlorophenyl)-6-(5-(1-(propyl-amino)-2-(methylthio) ethyl)-furan-2-yl) quinazolin-4-amine;
(R)-N-(4-(3-fluorobenzyl-oxy)-3-chlorophenyl)-6-(5-(1-(cyclopropyl-methylamino)-2-(methylthio)ethyl)-furan-2-yl) quinazolin-4-amine;
(R)-N-(4-(3-fluorobenzyl-oxy)-3-chlorophenyl)-6-(5-(1-(N,N-dimethyl-amino)-2-(methylthio)ethyl)-furan-2-yl) quinazolin-4-amine;
(R)-N-(4-(3-fluorobenzyloxy)-3-chlorophenyl)-6-(5-(1-(N,N-diethyl-amino)-2-(methylthio)ethyl)-furan-2-yl) quinazolin-4-amine;
(R)-N-(4-(3-fluorobenzyl-oxy)-3-chlorophenyl)-6-(5-(1-(N,N-dipropyl-amino)-2-(methylthio)ethyl)-furan-2-yl) quinazolin-4-amine;
(R)-N-(4-(3-fluorobenzyl-oxy)-3-chlorophenyl)-6-(5-(1-(N-methyl, N-ethyl-amino)-2-(methylthio)ethyl)-furan-2-yl) quinazolin-4-amine;
(R)-N-(4-(3-fluorobenzyl-oxy)-3-chlorophenyl)-6-(5-(1-(allyl-amino)-2-(methylthio) ethyl)-furan-2-yl) quinazolin-4-amine;
(R)-N-(4-(3-fluorobenzyl-oxy)-3-chlorophenyl)-6-(5-(1-(propargyl-amino)-2-(methylthio)ethyl)-furan-2-yl) quinazolin-4-amine;
(R)-N-(4-(3-fluorobenzyl-oxy)-3-chlorophenyl)-6-(5-(1-(methyl-amino)-2-(methylsulfinyl)ethyl)-furan-2-yl) quinazolin-4-amine;
(R)-N-(4-(3-fluorobenzyl-oxy)-3-chlorophenyl)-6-(5-(1-(ethyl-amino)-2-(methylsulfinyl)ethyl)-furan-2-yl) quinazolin-4-amine;
(R)-N-(4-(3-fluorobenzyl-oxy)-3-chlorophenyl)-6-(5-(1-(propyl-amino)-2-(methylsulfinyl)ethyl)-furan-2-yl) quinazolin-4-amine;
(R)-N-(4-(3-fluorobenzyl-oxy)-3-chlorophenyl)-6-(5-(1-(cyclopropyl-methyl-amino)-2-(methylsulfinyl)ethyl)-furan-2-yl) quinazolin-4-amine;
(R)-N-(4-(3-fluorobenzyl-oxy)-3-chlorophenyl)-6-(5-(1-(N,N-dimethyl-amino)-2-(methylsulfinyl)ethyl)-furan-2-yl)quinazolin-4-amine;
(R)-N-(4-(3-fluorobenzyl-oxy)-3-chlorophenyl)-6-(5-(1-(N,N-diethyl-amino)-2-(methylsulfinyl)ethyl)-furan-2-yl) quinazolin-4-amine;
(R)-N-(4-(3-fluorobenzyl-oxy)-3-chlorophenyl)-6-(5-(1-(N,N-dipropyl-amino)-2-(methylsulfinyl)ethyl)- furan-2-yl) quinazolin-4-amine;
(R)-N-(4-(3-fluorobenzyloxy)-3-chlorophenyl)-6-(5-(1-(N-methyl, N-ethyl-amino)-2-(methylsulfinyl)ethyl)-furan-2-yl) quinazolin-4-amine;
(R)-N-(4-(3-fluorobenzyl-oxy)-3-chlorophenyl)-6-(5-(1-(allyl-amino)-2-(methylsulfinyl) ethyl)-furan-2-yl) quinazolin-4-amine;
(R)-N-(4-(3-fluorobenzyl-oxy)-3-chlorophenyl)-6-(5-(1-(propargyl-amino)-2-(methylsulfinyl)ethyl)-furan-2-yl) quinazolin-4-amine;
(S)-N-(4-(3-fluorobenzyl-oxy)-3-chlorophenyl)-6-(5-(1-(amino)-2-(methylthio)ethyl)-furan-2-yl) quinazolin-4-amine;
(S)-N-(4-(3-fluorobenzyl-oxy)-3-chlorophenyl)-6-(5-(1-(methyl-amino)-2-(methylthio) ethyl)-furan-2-yl) quinazolin-4-amine;
(S)-N-(4-(3-fluorobenzyl-oxy)-3-chlorophenyl)-6-(5-(1-(ethyl-amino)-2-(methylthio) ethyl)-furan-2-yl) quinazolin-4-amine;
(S)-N-(4-(3-fluorobenzyl-oxy)-3-chlorophenyl)-6-(5-(1-(propyl-amino)-2-(methylthio) ethyl)-furan-2-yl) quinazolin-4-amine;
(S)-N-(4-(3-fluorobenzyl-oxy)-3-chlorophenyl)-6-(5-(1-(cyclopropyl-methylamino)-2-(methylthio)ethyl)-furan-2-yl) quinazolin-4-amine;
(S)-N-(4-(3-fluorobenzyl-oxy)-3-chlorophenyl)-6-(5-(1-(N,N-dimethyl-amino)-2-(methylthio)ethyl)-furan-2-yl) quinazolin-4-amine;
(S)-N-(4-(3-fluorobenzyl-oxy)-3-chlorophenyl)-6-(5-(1-(N,N-diethyl-amino)-2-(methylthio)ethyl)-furan-2-yl) quinazolin-4-amine;
(S)-N-(4-(3-fluorobenzyloxy)-3-chlorophenyl)-6-(5-(1-(N,N-dipropyl-amino)-2-(methylthio)ethyl)-furan-2-yl) quinazolin-4-amine;
(S)-N-(4-(3-fluorobenzyl-oxy)-3-chlorophenyl)-6-(5-(1-(N-methyl, N-ethyl-amino)-2-(methylthio)ethyl)-furan-2-yl) quinazolin-4-amine;
(S)-N-(4-(3-fluorobenzyl-oxy)-3-chlorophenyl)-6-(5-(1-(allyl-amino)-2-(methylthio) ethyl)-furan-2-yl) quinazolin-4-amine;
(S)-N-(4-(3-fluorobenzyl-oxy)-3-chlorophenyl)-6-(5-(1-(propargyl-amino)-2-(methylthio)ethyl)-furan-2-yl) quinazolin-4-amine;
(S)-N-(1-(3-fluorobenzyl)-1H-indazol-5-yl)-6-(5-(1-(methyl-amino)-2-(methylthio) ethyl)-furan-2-yl) quinazolin-4-amine;
(S)-N-(1-(3-fluorobenzyl)-1H-indazol-5-yl)-6-(5-(1-(ethyl-amino)-2-(methylthio)ethyl)-furan-2-yl) quinazolin-4-amine;
(S)-N-(1-(3-fluorobenzyl)-1H-indazol-5-yl)-6-(5-(1-(propyl-amino)-2-(methylthio)ethyl) -furan-2-yl) quinazolin-4-amine;
(S)-N-(1-(3-fluorobenzyl)-1H-indazol-5-yl)-6-(5-(1-(N,N-dimethyl-amino)-2-(methylthio)ethyl)-furan-2-yl) quinazolin-4-amine;
(S)-N-(1-(3-fluorobenzyl)-1H-indazol-5-yl)-6-(5-(1-(cyclopropyl-methylamino)-2-(methylthio)ethyl)-furan-2-yl) quinazolin-4-amine;
(S)-N-(1-(3-fluorobenzyl)-1H-indazol-5-yl)-6-(5-(1-(N-methyl, N-ethylamino)-2-(methylthio)ethyl)-furan-2-yl) quinazolin-4-amine;
(S)-N-(1-(3-fluorobenzyl)-1H-indazol-5-yl)-6-(5-(1-(allyl-amino)-2-(methylthio)ethyl)-furan-2-yl) quinazolin-4-amine;
(S)-N-(1-(3-fluorobenzyl)-1H-indazol-5-yl)-6-(5-(1-(propargyl-amino)-2-(methylthio) ethyl)-furan-2-yl) quinazolin-4-amine;
(S)-N-(4-(3-fluorobenzyl-oxy)-3-chlorophenyl)-6-(5-(1-(methyl-amino)-2-(methylsulfinyl)ethyl)-furan-2-yl) quinazolin-4-amine;
(S)-N-(4-(3-fluorobenzyl-oxy)-3-chlorophenyl)-6-(5-(1-(ethyl-amino)-2-(methylsulfinyl)ethyl)-furan-2-yl) quinazolin-4-amine;
(S)-N-(4-(3-fluorobenzyl-oxy)-3-chlorophenyl)-6-(5-(1-(propyl-amino)-2-(methylsulfinyl)ethyl)-furan-2-yl) quinazolin-4-amine;
(S)-N-(4-(3-fluorobenzyl-oxy)-3-chlorophenyl)-6-(5-(1-(cyclopropyl-methylamino)-2-(methylsulfinyl)ethyl)-furan-2-yl) quinazolin-4-amine;
(S)-N-(4-(3-fluorobenzyl-oxy)-3-chlorophenyl)-6-(5-(1-(N,N-dimethyl-amino)-2-(methylsulfinyl)ethyl)-furan-2-yl) quinazolin-4-amine;
(S)-N-(4-(3-fluorobenzyl-oxy)-3-chlorophenyl)-6-(5-(1-(N,N-diethyl-amino)-2-(methylsulfinyl)ethyl)-furan-2-yl) quinazolin-4-amine;
(S)-N-(4-(3-fluorobenzyl-oxy)-3-chlorophenyl)-6-(5-(1-(N,N-dipropyl-amino)-2-(methylsulfinyl)ethyl)-furan-2-yl) quinazolin-4-amine;
(S)-N-(4-(3-fluorobenzyl-oxy)-3-chlorophenyl)-6-(5-(1-N-methyl, N-ethyl-amino)-2-(methylsulfinyl)ethyl)furan-2-yl) quinazolin-4-amine;
(S)-N-(4-(3-fluorobenzyl-oxy)-3-chlorophenyl)-6-(5-(1-(allyl-amino)-2-(methylsulfinyl)ethyl)-furan-2-yl) quinazolin-4-amine; and
(S)-N-(4-(3-fluorobenzyl-oxy)-3-chlorophenyl)-6-(5-(1-(propargyl-amino)-2-(methylsulfinyl)ethyl)-furan-2-yl) quinazolin-4-amine.

13. Pharmaceutical composition comprising a therapeutically effective amount of the compounds of any one of claims 1 to 7 or pharmaceutically acceptable salts and pharmaceutically acceptable carriers thereof.

14. Use of the compounds of general formula (I) in any one of claims 1-7 or pharmaceutically acceptable salts thereof in the preparation of a medicament for the treatment of diseases associated with regulating c-erbB-2 and / or EGF-R protein tyrosine kinase activity.

15. Use of the pharmaceutical composition of claim 13 in the preparation of a medicament for the treatment of diseases associated with regulating c-erbB-2 and /or EGF-R protein tyrosine kinase activity.

16. Use of claim 14 or 15, wherein the disease is malignant tumor or psoriasis.
